(19) Europäisches Patentamt
European Patent Office
Office européen des brevets

(11) EP 1 291 007 A1

(12) **EUROPÄISCHE PATENTANMELDUNG**

(43) Veröffentlichungstag:
**12.03.2003 Patentblatt 2003/11**

(51) Int Cl.[7]: **A61K 7/42**

(21) Anmeldenummer: **02019206.8**

(22) Anmeldetag: **27.08.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **07.09.2001 DE 10143964**

(71) Anmelder: **BASF AKTIENGESELLSCHAFT**
**67056 Ludwigshafen (DE)**

(72) Erfinder:
- **Heidenfelder, Thomas, Dr.**
  **67125 Dannstadt (DE)**
- **Wünsch, Thomas, Dr.**
  **67346 Speyer (DE)**
- **Andre, Valerie, Dr.**
  **67063 Ludwigshafen (DE)**

(54) **Emulgatorarme oder emulgatorfreie Systeme vom Typ Öl-in-Wasser mit einem Gehalt an Stabilisatoren und einem aminosubstituierten Hydroxybenzophenon**

(57) Kosmetische oder dermatologische Zubereitungen, die feindisperse Systeme vom Typ Öl-in-Wasser darstellen, enthaltend

a) eine Ölphase,

b) eine Wasserphase,

c) einen oder mehrere Stabilisatoren,

d) höchstens 2,00 Gew.-% eines oder mehrerer Emulgatoren und

e) ein aminosubstituiertes Hydroxybenzophenon der Formel I

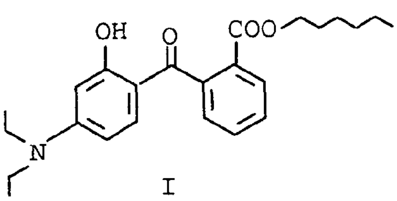

I

EP 1 291 007 A1

## Beschreibung

Die vorliegende Erfindung betrifft emulgatorfreie oder emulgatorarme feindisperse Systeme vom Typ Öl-in Wasser, bevorzugt als kosmetische oder dermatologische Zubereitungen.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z.B. Milch). Der Grundcharakter einer O/W-Emulsion ist durch das Wasser geprägt. Bei einer Wasser-in-Öl-Emulsion (W/O-Emulsion, z.B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere) und nichtionische untergliedert werden:

- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.

- Wichtige Vertreter der kationischen Emulgatoren sind die quaternären Ammonium-Verbindungen.

- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Esterund Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z.B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar.

Kosmetische Zubereitungen werden im wesentlichen zur Hautpflege benutzt. Hautpflege im kosmetischen Sinn ist in erster Linie, dass die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Kosmetische Zubereitungen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Medizinische topische Zusammensetzungen enthalten in der Regel ein oder mehrere Medikamente in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittelund Arzneimittelgesetz).

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen.

So ist beispielsweise bekannt, dass bestimmte Lichtdermatosen durch gewisse Emulgatoren, aber auch durch verschiedene Fette und gleichzeitige Exposition von Sonnenlicht ausgelöst werden. Solche Lichtdermatosen werden auch "Mallorca-Akne" genannt. Es hat daher nicht an Versuchen gefehlt, die Menge an üblichen Emulgatoren auf ein Minimum, im Idealfall sogar vollständig zu reduzieren.

Der Stand der Technik kennt neben den beschriebenen Emulsionen weitere emulgatorfreie, feindisperse kosmetische oder dermatologische Zubereitungen, die im allgemeinen als Hydrodispersionen bezeichnet werden. Hydrodispersionen stellen Dispersionen einer flüssigen, halbfesten oder festen inneren (diskontinuierlichen) Lipidphase in einer äußeren wässrigen (kontinuierlichen) Phase dar.

Bei Hydrodispersionen einer flüssigen Lipidphase in einer äußeren wässrigen Phase kann die Stabilität beispielsweise dadurch gewährleistet werden, dass in der wässrigen Phase ein Gelgerüst aufgebaut wird, in welchem die Lipidtröpfchen stabil suspendiert sind. Die Deutsche Offenlegungsschrift 44 25 268 beschreibt stabile feindisperse, emulgatorfreie kosmetische oder dermatologische Zubereitungen vom Typ Öl-in-Wasser, die neben einer Ölund einer Wasserphase einen oder mehrere Verdicker aus der Gruppe der Acrylsäurepolymere, Polysaccharide und deren Alkylether enthalten, wobei für diese Verdicker eine Grenzflächenspannungserniedrigung nicht messbar sein darf.

Basierend auf ähnlichen Hydrodispersionen werden in der Deutschen Offenlegungsschrift 43 03 983 kosmetische oder dermatologische Lichtschutzformulierungen offenbart, die im wesentlichen frei von Emulgatoren sind, wobei in die Lipidphase der Hydrodispersion anorganische Mikropigmente eingearbeitet sind, die als UV-Filtersubstanzen dienen.

Obwohl einerseits eine Vielzahl an Möglichkeiten besteht, gänzlich emulgatorfreie Zubereitungen zu konzipieren, ist andererseits ein gewisser geringer Emulgatorgehalt gelegentlich nicht ganz unerwünscht, da die Abwesenheit von Emulgatoren bei der Einarbeitung vieler Rohstoffe technische Schwierigkeiten bereiten.

Aufgabe der vorliegenden Erfindung war es, den Stand der Technik um kosmetische oder dermatologische Zubereitungen zu bereichern, mittels derer unter Duldung eines geringen Gehaltes an Emulgatoren stabile Zubereitungen mit gutem Hautgefühl erhalten werden.

Erstaunlicherweise wird diese Aufgabe gelöst durch kosmetische oder dermatologische Zubereitungen, die feindisperse Systeme vom Typ Öl-in-Wasser darstellen, enthaltend

a) eine Ölphase,

b) eine Wasserphase,

c) einen oder mehrere Stabilisatoren,

d) höchstens 2,00 Gew.-% eines oder mehrerer Emulgatoren und

e) ein aminosubstituiertes Hydroxybenzophenon der Formel I

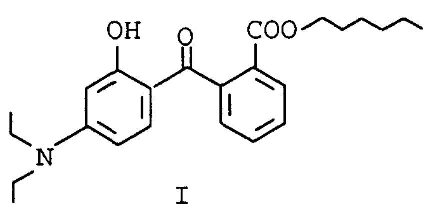

I

sowie gegebenenfalls weitere kosmetische oder pharmazeutische Hilfs-, Zusatz- und/oder Wirkstoffe.

Es ist erfindungsgemäß besonders vorteilhaft, wenn die Zubereitungen emulgatorfrei sind, höchstens aber 0,1 bis 2,00, bevorzugt 0,5 bis 1,5 Gew.-%, bezogen auf das Gesamtgewicht der Zubereitungen, an einem oder mehreren Emulgatoren enthalten.

Die in den erfindungsgemäßen Zubereitungen verwendeten Stabilisatoren sind vorzugsweise Vertreter aus der Stoffklasse der Hydrokolloide.

"Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Nebenund Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wässrigen Medien ist. Voraussetzung dafür ist, dass diese Polymere über eine für die Wasserlöslichkeit ausreichende Anzahl an hydrophilen Gruppen besitzen und nicht zu stark vernetzt sind.

Die hydrophilen Gruppen können nichtionischer, anionischer oder kationischer Natur sein, beispielsweise wie folgt:

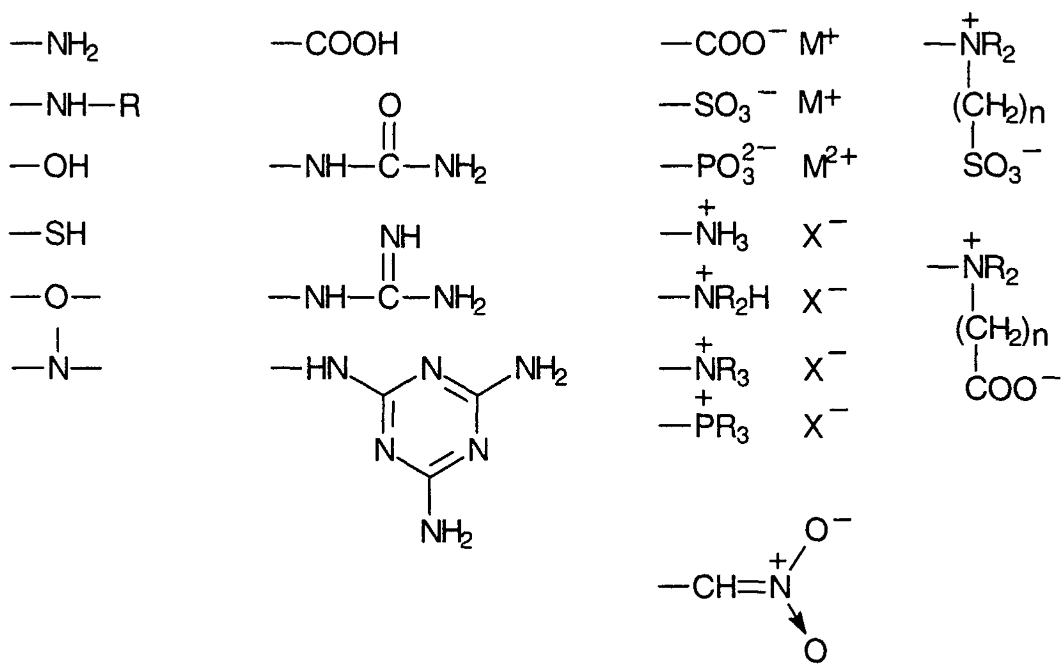

Die Gruppe der kosmetisch und dermatologisch relevanten Hydrokolloide lässt sich wie folgt einteilen in:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein;
- organische, abgewandelte Naturstoffe, wie z.B. Carboxymethylcellulose und andere Celluloseether, Hydroxyethyl- und -propylcellulose und mikrokristalline Cellulose;
- organische, vollsynthetische Verbindungen, wie z.B. Polyacryl- und Polymethacryl-Verbindungen, Vinylpolymere, Polycarbonsäuren, Polyether, Polyimine, Polyamide, Polyurethane;
- anorganische Verbindungen, wie z.B. Polykieselsäuren, Tonmineralien wie Montmorillonite, Zeolithe, Kieselsäuren.

Vorteilhafte Stabilisatoren sind Ethylcellulosen. Ethylcellulosen sind gekennzeichnet durch folgende Struktur:

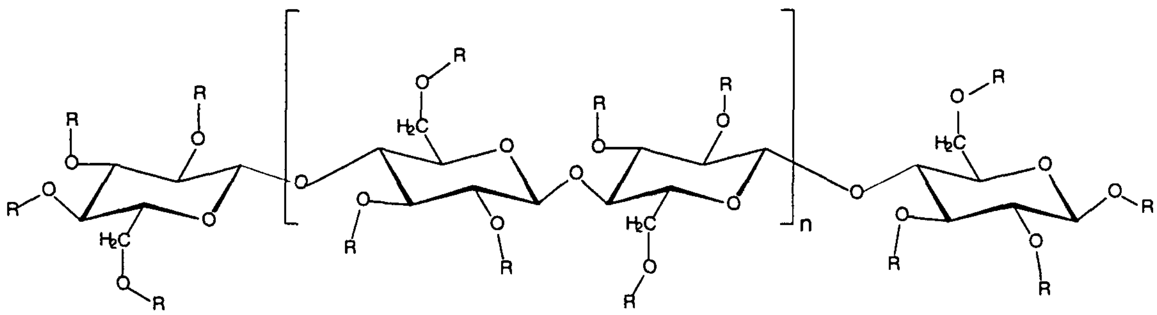

Dabei kann R sowohl Ethylgruppen wie auch Wasserstoffatome darstellen.

Der Ethylierungsgrad der Ethylcellulose beträgt vorteilhaft 2,0 bis 3,0, entsprechend 40 bis 55 %, bevorzugt 48,0 bis 49,5 % Ethylierung. Die mittlere Molmasse ist bevorzugt so zu wählen, dass die Viskosität einer 5 %-igen Lösung in einem Gemisch aus 80 Teilen Toluol und 20 Teilen Ethanol bei 25°C 3 bis 110 mPas, bevorzugt 9 bis 11 mPas, beträgt. Insbesondere vorteilhaft ist die mittlere Molmasse 100000 und 400000 g/mol.

Bevorzugt beträgt der Gehalt an Ethylcellulose in den erfindungsgemäßen Zubereitungen 0,1 bis 10 Gew.-%, bezogen auf das Gesamtgewicht der Zubereitungen. Solche Produkte sind beispielsweise erhältlich unter der Handelsbezeichnung ETHOCEL® Standard 10 Premium (Dow Chemicals).

Mikrokristalline Cellulose ist ein vorteilhaftes Hydrokolloid im Sinne der vorliegenden Erfindung. Sie ist beispielsweise von der "FMC Corporation Food and Pharmaceutical Products" unter der Handelsbezeichnung Avicel® erhältlich. Ein besonders vorteilhaftes Produkt im Sinne der vorliegenden Erfindung ist der Typ Avicel® RC-591, bei dem es sich um modifizierte mikrokristalline Cellulose handelt, die sich zu 89% aus mikrokristalliner Cellulose und zu 11% aus Natrium Carboxymethyl Cellulose zusammensetzt. Weitere Handelsprodukte dieser Rohstoffklasse sind Avicel® RC/ CL, Avicel® CE-15, Avicel® 500.

Weitere vorteilhaft verwendete Hydrokolloide sind beispielsweise Methylcellulosen, als welche die Methylether der Cellulose bezeichnet werden. Sie zeichnen sich durch die folgende Strukturformel aus,

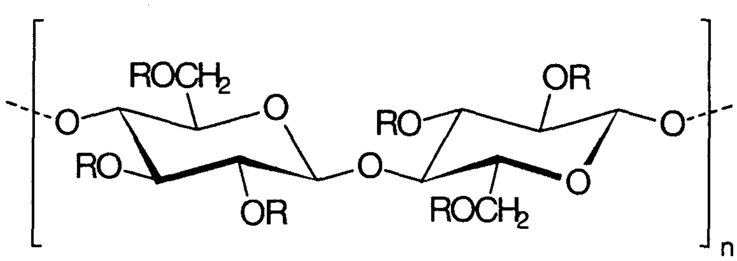

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, die beispielsweise unter der Handelsbezeichnung Methocel® E4M bei der Dow Chemical Comp. erhältlich sind.

Erfindungsgemäß ferner vorteilhaft ist Natriumcarboxymethylcellulose, das Natrium-Salz des Glykolsäureethers der Cellulose, für welches R in obiger Strukturformel ein Wasserstoff und/oder $CH_2$-COONa darstellen kann. Besonders bevorzugt ist die unter der Handelsbezeichnung Natrosol® Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2x106 bis 24x106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans.

Ein weiterer vorteilhafter Gelbildner im Sinne der vorliegenden Erfindung ist ferner Carrageen, ein gelbildender und ähnlich wie Agar aufgebauter Extrakt aus nordatlantischen, zu den Florideen zählenden Rotalgen (Chondrus crispus und Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100.000 bis 800.000 und einem Sulfat-Gehalt von ca. 25 %. Carrageen, das in warmem Wasser sehr leicht löslich ist, bildet beim Abkühlen ein thixotropes Gel, selbst wenn der Wassergehalt 95-98 % beträgt. Die Festigkeit des Gels wird durch die Doppelhelix-Struktur des Carrageens bewirkt.

Beim Carrageenan unterscheidet man drei Hauptbestandteile:

Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt und in kaltem Wasser leicht löslich. Das aus D-Galactose-4-sulfat in 1,3-Bindung und 3,6-Anhydro-α-D-galactose-2-sulfat in 1,4-Bindung aufgebaute τ-Carrageenan ist sowohl wasserlöslich als auch gelbildend. Auch die Art vorhandener Kationen (K+, NH4+, Na+, Mg2+, Ca2+) beeinflusst die Löslichkeit der Carrageene.

Polyacrylate sind im Sinne der vorliegenden Erfindung ebenfalls bevorzugt verwendete Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eine eingetragene Marke der B.F. Goodrich Company) gewählt werden. Insbesondere zeichnen sich das oder die erfindungsgemäß vorteilhaften Acrylat-Alkylacrylat-Copolymere durch die folgende Struktur aus:

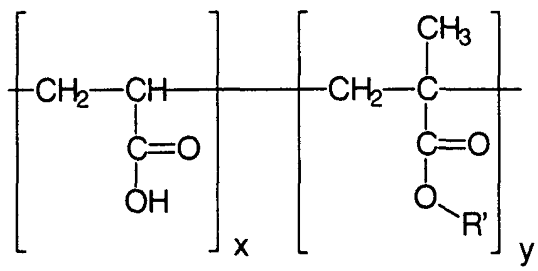

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelsbezeichnungen Carbopol® 1382, Carbopol® 981 und Carbopol® 5984 von der B.F. Goodrich Company erhältlich sind, ganz besonders bevorzugt sind Polyacrylate aus der Gruppe der Carbopole der Typen 980, 981, 1382, 2984, 5984 sowie Carbomer 2001.

Ferner vorteilhaft sind Copolymere aus $C_{10}$-$C_{30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind ebenfalls Verbindungen, die die INCI-Bezeichnung "Acrylates/$C_{10}$-$C_{30}$ Alkyl Acrylate Crosspolymer" tragen. Insbesondere vorteilhaft sind die unter den Handelsbezeichnungen Pemulen® TR1 und Pemulen® TR2 bei der B.F. Goodrich Company erhältlichen Polymere.

Vorteilhaft sind Verbindungen, die die INCI-Bezeichnung Ammoniumacryloyldimethyltaurate/Vinylpyrrolidoncopolymere tragen.

Erfindungsgemäß vorteilhaft weisen das oder die Ammoniumacryloyldimethyltaurate/Vinylpyrrolidoncopolymere die Summenformel $[C_7H_{16}N_2SO_4]_n$ $[C_6H_9NO]_m$ auf, die der folgenden statistischen Struktur entspricht

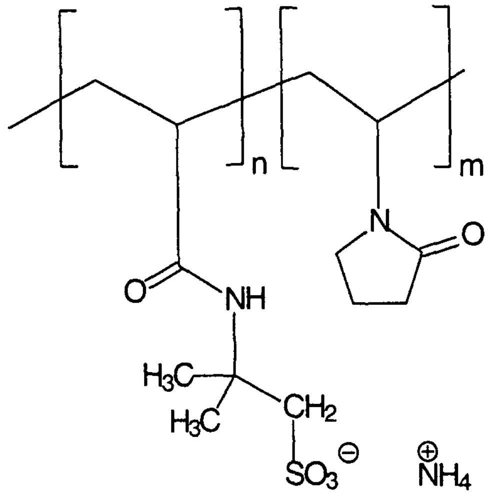

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH.

Vorteilhaft sind ferner Copolymere/Crosspolymere umfassend Acryloyldimethyl Taurate, wie beispielsweise Simugel® EG oder Simugel® EG von der Gesellschaft Seppic S.A.

Weitere erfindungsgemäß zu verwendende vollsynthetische Hydrokolloide sind:

A. in Wasser lösliche oder dispergierbare anionische Polyurethane, welche vorteilhaft erhältlich sind aus

Aa) mindestens einer Verbindung, die zwei oder mehrere aktive Wasserstoffatome pro Moleküle enthält,

Ab) mindestens einem Säure- oder Salzgruppen enthaltenden Diol und

Ac) mindestens einem Diisocyanat.

Bei der Komponente Aa) handelt es sich insbesondere um Diole, Aminoalkohole, Diamine, Polyesterole, Polyetherole mit einem zahlenmittleren Molekulargewicht von jeweils bis zu 3000 oder deren Mischungen, wobei bis zu 3 Mol-% der genannten Verbindungen durch Triole oder Triamine ersetzt sein können. Bevorzugt sind Diole und Polyesterdiole. Insbesondere umfasst die Komponente Aa) mindestens 50 Gew.-%, bezogen auf das Gesamtgewicht der Komponente Aa), eines Polyesterdiols. Als Polyesterdiole kommen alle diejenigen in Betracht, die üblicherweise zur Herstellung von Polyurethanen eingesetzt werden, insbesondere Umsetzungsprodukte aus Phthalsäure und Diethylenglycol, Isophthalsäure und 1,4-Butandiol, Isophthalsäure/Adipinsäure und 1,6-Hexandiol sowie Adipinsäure und Ethylenglycol oder 5-$NaSO_3$-Isophthalsäure, Phthalsäure, Adipinsäure und 1,6-Hexandiol.

Brauchbare Diole sind z.B. Ethylenglycol, Propylenglycol, Butylenglycol, Neopentylglycol, Polyetherole, wie Polyethylenglycole mit Molekulargewichten bis zu 3000, Blockcopolymerisate aus Ethylenoxid und Propylenoxid mit zahlenmittleren Molekulargewichten von bis zu 3000 oder Blockcopolymerisate aus Ethylenoxid, Propylenoxid und Butylenoxid, die die Alkylenoxideinheiten statistisch verteilt oder in Form von Blöcken einpolymerisiert enthalten. Bevorzugt sind Ethylenglycol, Neopentylglycol, Di-, Tri-, Tetra-, Penta oder Hexaethylenglyol. Brauchbare Diole sind außerdem Poly($\alpha$-hydroxycarbonsäure)diole.

Geeignete Aminoalkohole sind z.B. 2-Aminoethanol, 2-(N-Methylamino)ethanol, 3-Aminopropanol oder 4-Aminobutanol.

Geeignete Diamine sind z.B. Ethylendiamin, Propylendiamin, 1,4-Diaminobutan und 1,6-Diaminohexan sowie $\alpha,\omega$-Diamine, die durch Aminierung von Polyalkylenoxiden mit Ammoniak herstellbar sind.

Bei der Komponente Ab) handelt es sich insbesondere um Dimethylolpropansäure oder Verbindungen der Formeln

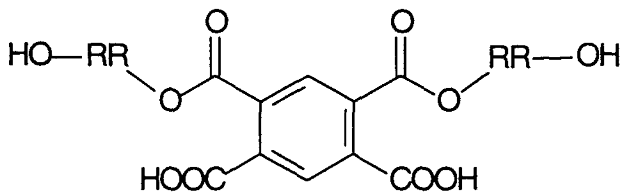

bzw.

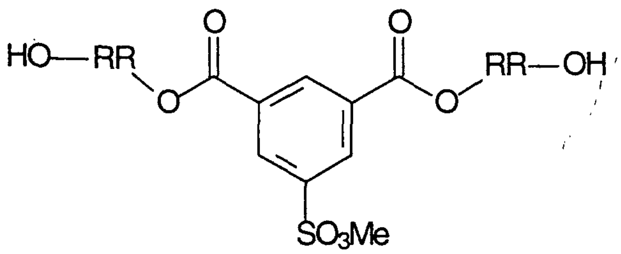

worin RR jeweils für eine $C_2$-$C_{18}$-Alkylengruppe steht Me Na oder K bedeutet.

Bei der Komponente Ac) handelt es sich insbesondere um Hexamethylendiisocyanat, Isophorondiisocyanat, Methyldiphenylisocyanat (MDI) und/oder Toluylendiisocyanat.

Die Polyurethane sind dadurch erhältlich, dass man die Verbindungen der Gruppen Aa) und Ab) unter einer Inertgasatmosphäre in einem inerten Lösemittel bei Temperaturen von 70 bis 130°C mit den Verbindungen der Gruppe Ac) umsetzt. Diese Umsetzung kann gegebenenfalls in Gegenwart von Kettenverlängerern durchgeführt werden, um Polyurethane mit höheren Molekulargewichten herzustellen. Wie bei der Herstellung von Polyurethanen üblich, werden die Komponenten [(Aa)+(Ab)]:Ac vorteilhaft im molaren Verhältnis von 0,8 bis 1,1 : 1 eingesetzt. Die Säurezahl der Polyurethane wird von der Zusammensetzung und der Konzentration der Verbindungen der Komponente (Ab) in der Mischung aus den Komponenten (Aa) und (Ab) bestimmt.

Die Polyurethane haben K-Werte nach H. Fikentscher (bestimmt in 0,1 gew.-%igen Lösungen in N-Methylpyrrolidon bei 25°C und pH 7) von 15 bis 100, vorzugsweise 25 bis 50.

Der auch als Eigenviskosität bezeichnete K-Wert ist ein über Viskositätsmessungen von Polymerlösungen einfach zu bestimmender und daher im technischen Bereich häufig benutzter Parameter zur Charakterisierung von Polymeren.

Die Säuregruppen enthaltenden Polyurethane sind nach Neutralisation (teilweise oder vollständig) wasserlöslich bzw. ohne Zuhilfenahme von Emulgatoren dispergierbar. In aller Regel weisen die Salze der Polyurethane eine bessere Wasserlöslichkeit oder Dispergierbarkeit in Wasser auf als die nicht neutralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Ammoniak und Amine verwendet werden. Besonders haben sich zur Neutralisation der Säuregruppen enthaltenden Polyurethane 2-Amino-2-Methylpropanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z.B. Mischungen aus Natronlauge und Triisopropanolamin. Die Neutralisation kann je nach Anwendungszweck partiell z.B. zu 20 bis 40 % oder vollständig, d.h. zu 100 % erfolgen.

Diese Polymere und ihre Herstellung sind in DE-A-42 25 045 näher beschrieben, auf die hiermit in vollem Umfang Bezug genommen wird.

B. In Wasser lösliche oder dispergierbare, kationische Polyurethane und Polyharnstoffe aus

Ba) mindestens einem Diisocyanat, welches bereits vorher mit einer oder mehreren Verbindungen, die zwei oder mehrere aktive Wasserstoffatome pro Moleküle enthalten, umgesetzt worden sein kann, und
Bb) mindestens einem Diol, primären oder sekundären Aminoalkohol, primären oder sekundärem Diamin oder primären oder sekundären Triamin mit einem oder mehreren tertiären, quaternären oder protonierten tertiären Aminostickstoffatomen.

Bevorzugte Diisocyanate sind wie oben unter A) angegeben. Verbindungen mit zwei oder mehreren aktiven Wasserstoffatomen sind Diole, Aminoalkohole, Diamine, Polyesterole. Polyamiddiamine und Polyetherole. Geeignete Verbindungen dieser Art sind wie oben unter A) angegeben.

Die Herstellung der Polyurethane erfolgt wie oben unter A) beschrieben. Geladene kationische Gruppierungen lassen sich aus den vorliegenden tertiären Aminostickstoffatomen entweder durch Protonierung, z.B. mit Carbonsäuren wir Milchsäure, oder durch Quaternisierung, z.B. mit Alkylierungsmitteln wie $C_1$ bis $C_4$-Alkylhalogeniden oder -sulfaten in den Polyharnstoffen erzeugen. Beispiele solcher Alkylierungsmittel sind Ethylchlorid, Ethylbromid, Methylchlorid, Methylbromid, Dimethylsulfat und Diethylsulfat.

Diese Polymere und ihre Herstellung sind in der DE-A-42 41 118 näher beschrieben, auf die hiermit in vollem Umfang Bezug genommen wird.

C. Lineare Polyurethane mit Carboxylatgruppen aus

Ca) einer 2,2-Hydroxymethyl-substituierten Carbonsäure der Formel

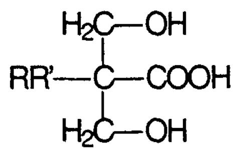

worin RR' für ein Wasserstoffatom oder eine $C_1$-$C_{20}$-Alkylgruppe steht, die in einer Menge verwendet wird, welche ausreicht, dass in dem Polyurethan 0,35 bis 2,25 Milliäquivalente Carboxylgruppen pro g Polyurethan vorhanden sind,

Cb) 10 bis 90 Gew.-%, bezogen auf das Gewicht des Polyurethans, einer oder mehrerer organischer Verbindungen mit nicht mehr als zwei aktiven Wasserstoffatomen und

Cc) einem oder mehreren organischen Diisocyanaten.

Die im Polyurethan enthaltenden Carboxylgruppen werden abschließend mit einer geeigneten Base zumindest teilweise neutralisiert. Diese Polymere und ihre Herstellung sind in der EP-A-619 111 beschrieben, auf die hiermit in vollem Umfang Bezug genommen wird.

D. Carboxylhaltige Polykondensationsprodukte aus Anhydriden von Tri- oder Tetracarbonsäuren und Diolen, Diaminen oder Aminoalkoholen (Polyester, Polyamide oder Polyesteramide). Diese Polymere und ihre Herstellung sind in der DE-A-42 24 761 näher beschrieben, auf die hiermit in vollem Umfang Bezug genommen wird.

E. Polyacrylate und Polymethacrylate, wie sie in den DE-A-43 14 305, 36 27 970 und 29 17 504 näher beschrieben sind. Auf diese Publikationen wird hiermit in vollem Umfang Bezug genommen.

Die erfindungsgemäß zur Anwendung kommenden Polymere besitzen vorzugsweise einen K-Wert von 15 bis 100, bevorzugt 25 bis 50. Die Polymere sind in dem erfindungsgemäßen Mittel im allgemeinen in einer Menge im Bereich von 0,2 bis 20 Gew.-%, bezogen auf das Gesamtgewicht des Mittels, enthalten. Das Salz kommt in einer zur Verbesserung der Austauschbarkeit der Polymeren wirksamen Menge zur Anwendung. Im allgemeinen setzt man das Salz in einer Menge von 0,02 bis 10 Gew.-%, vorzugsweise 0,05 bis 5 Gew.-% und insbesondere 0,1 bis 3 Gew.-%, bezogen auf das Gesamtgewicht des Mittels, ein.

Als anorganische Verdickungsmittel können beispielsweise Vertreter aus der Gruppe der modifizierten oder unmodifizierten, natürlich vorkommenden oder synthetischen Schichtsilikate gewählt werden.

Es ist zwar durchaus günstig, reine Komponenten einzusetzen, es können jedoch auch in vorteilhafter Weise, Gemische verschiedener modifizierter und/oder unmodifizierter Schichtsilicate in den erfindungsgemäßen Zusammensetzungen enthalten sein.

Unter Schichtsilicaten, welche auch Phyllosilicate genannt werden, sind im Rahmen dieser Anmeldung Silicate und Alumosilicate zu verstehen, in welchen die Silicat- bzw. Aluminateinheiten über drei Si-O- oder Al-O- Bindungen untereinander verknüpft sind und eine gewellte Blatt- oder Schichtenstruktur ausbilden. Die vierte Si-O- bzw. Al-O- Valenz wird durch Kationen abgesättigt. Zwischen den einzelnen Schichten bestehen schwächere elektrostatische Wechselwirkungen, z.B. Wasserstoffbrückenbindungen. Das Schichtgefüge indessen ist weitgehend durch starke, kovalente Bindungen geprägt.

Die Stöchiometrie der Blattsilicate ist ($Si_2O_5^{2-}$) für reine Silicatstrukturen und ($Al_mSi^{2-}{}_mO_5^{(2+m)-}$) für Alumosilicate.

m ist eine Zahl größer als Null und kleiner als 2.

Liegen keine reinen Silicate sondern Alumosilicate vor, ist dem Umstande Rechnung zu tragen, dass jede durch $A1^{3+}$ ersetzte $Si^{4+}$ - Gruppe ein weiteres einfach geladenes Kation zur Ladungsneutralisierung erfordert.

Die Ladungsbilanz wird bevorzugt durch $H^+$, Alkali- oder Erdalkalimetallionen ausgeglichen. Auch Aluminium als Gegenion ist bekannt und vorteilhaft. Im Gegensatz zu den Alumosilicaten werden diese Verbindungen Aluminiumsilicate genannt. Auch "Aluminiumalumosilicate", in welchen Aluminium sowohl im Silicatnetz, als auch als Gegenion vorliegt, sind bekannt und für die vorliegende Erfindung gegebenenfalls von Vorteil.

Schichtsilicate sind in der Literatur gut dokumentiert, z.B. im "Lehrbuch der Anorganischen Chemie", A.F. Hollemann, E. Wiberg und N. Wiberg, 91.-100. Aufl., Walter de Gruyter - Verlag 1985, passim, sowie "Lehrbuch der Anorganischen Chemie", H. Remy, 12. Aufl., Akademische Verlagsgesellschaft, Leipzig 1965, passim. Die Schichtenstruktur von Montmorillonit ist Römpps ChemieLexikon, Franckh'sche Verlagshandlung W. Keller & Co., Stuttgart, 8. Aufl., 1985, S. 2668 f., zu entnehmen.

Beispiele für Schichtsilicate sind:

| | |
|---|---|
| Montmorillonit | $Na_{0,33}((Al_{1,67}Mg_{0,33})\ (OH)_2(Si_4O_{10}))$ |
| oft vereinfacht: | $Al_2O_3{*}4SiO_2{*}H_2O{*}nH_2O$ bzw. |
| | $Al_2[(OH)_2/Si_4O_{10}] \cdot n\ H_2O$ |
| Kaolinit | $Al_2(OH)_4(Si_2O_5)$ |
| Ilit | $(K,H_3O)_y(Mg_3(OH)_2(Si_{4-y}Al_yO_{10}))$ bzw. |
| | $(K,H_3O)_y(Al_2(OH)_2(Si_{4-y}Al_yO_{10}))$ |
| | mit y = 0,7 - 0,9 |
| Beidellit | $(Ca,Na)_{0,3}(Al_2(OH)_2(Al_{0,5}Si_{3,5}O_{10}))$ |
| Nontronit | $Na_{0,33}(Fe_2(OH)_2(Al_{0,33}Si_{3,67}O_{10}))$ |
| Saponit | $(Ca,Na)_{0,33}((Mg,Fe)_3(OH)_2(Al_{0,33}Si_{3,67}O_{10}))$ |
| Hectorit | $Na_{0,33}((Mg,Li)_3(OH,F)_2(Si_4O_{10}))$ |

Montmorillonit stellt das Hauptmineral der natürlich vorkommenden Bentonite dar.

Sehr vorteilhafte anorganische Gelbildner im Sinne der vorliegenden Erfindung sind Aluminiumsilikate wie die Montmorillonite (Bentonite, Hectorite sowie deren Derivate wie Quaternium-18 Bentonit, Quaternium-18 Hectorite, Stearalkonium Bentonite bzw. Stearalkonium Hectorite) oder aber Magnesium-Aluminium-Silikate (Veegum®-Typen) sowie Natrium-Magnesium-Silikate (Laponite®-Typen)

Montmorillonite stellen zu den dioktaedrischen Smektiten gehörende Tonmineralien dar und sind in Wasser quellende, aber nicht plastisch werdende Massen. Die Schichtpakete in der Dreischicht-Struktur der Montmorillonite können durch reversible Einlagerung von Wasser (in der 2 bis 7 fachen Menge) u.a. Substanzen wie z.B. Alkoholen, Glykolen, Pyridin, $\alpha$-Picolin, Ammonium-Verbindungen, Hydroxy-Aluminosilicat-Ionen usw. aufquellen.

Die oben angegebene chemische Formel ist nur angenähert; da Montmorillonite ein großes Ionenaustausch-Vermögen besitzen, kann Al gegen Mg, $Fe^{2+}$, $Fe^{3+}$, Zn, Pb (z.B. aus Schadstoffen in Abwässern) Cr, auch Cu und andere ausgetauscht werden. Die daraus resultierende negative Ladung der Oktaeder-Schichten wird durch Kationen, insbesondere $Na^+$ (Natrium-Montmorillonit) und $Ca^{2+}$ (der Caicium-Montmorillonit ist nur sehr wenig quellfähig) in Zwischenschicht-Positionen ausgeglichen.

Im Sinne der vorliegenden Erfindung vorteilhafte synthetische Magnesiumsilikate bzw. Bentonite werden beispielsweise von Süd-Chemie unter der Handelsbezeichung Optigel® vertrieben.

Ein im Sinne der vorliegenden Erfindung vorteilhaftes Aluminiumsilikat wird beispielsweise von der R.T. Vanderbilt Comp., Inc., unter der Handelsbezeichnung Veegum® vertrieben. Die verschiedenen Veegum®-Typen, welche alle erfindungsgemäß vorteilhaft sind, zeichnen sich durch folgende Zusammensetzungen aus

| | (regular grade) | HV | K | HS | S-728 |
|---|---|---|---|---|---|
| $SiO_2$ | 55,5 | 56,9 | 64,7 | 69,0 | 65,3 |
| MgO | 13,0 | 13,0 | 5,4 | 2,9 | 3,3 |

(fortgesetzt)

| | (regular grade) | HV | K | HS | S-728 |
|---|---|---|---|---|---|
| $Al_2O_3$ | 8,9 | 10,3 | 14,8 | 14,7 | 17,0 |
| $Fe_2O_3$ | 1,0 | 0,8 | 1,5 | 1,8 | 0,7 |
| CaO | 2,0 | 2,0 | 1,1 | 1,3 | 1,3 |
| $Na_2O$ | 2,1 | 2,8 | 2,2 | 2,2 | 3,8 |
| $K_2O$ | 1,3 | 1,3 | 1,9 | 0,4 | 0,2 |
| Veraschungsverlust | 11,1 | 12,6 | 7,6 | 5,5 | 7,5 |

Diese Produkte quellen in Wasser unter Bildung viskoser Gele, welche alkalisch reagieren. Durch Organophilierung von Montmorillonit bzw. Bentoniten (Austausch der Zwischenschicht-Kationen gegen quaternäre Alkylammonium-Ionen) entstehen Produkte (Bentone), die bevorzugt zur Dispergierung in organischen Lösemitteln und Ölen, Fetten, Salben, Farben, Lacken und in Waschmitteln eingesetzt werden.

Bentone® ist eine Handelsbezeichnung für verschiedene neutrale und chemisch inerte Geliermittel, die aus langkettigen, organischen Ammoniumsalzen und speziellen Montmorillonit-Sorten aufgebaut sind. Bentone quellen in organischen Medien und bringen diese zum Quellen. Die Gele sind in verdünnten Säuren und Alkalien beständig, bei längerer Berührung mit starken Säuren und Alkalien verlieren sie ihre Geliereigenschaften jedoch teilweise. Aufgrund ihres organophilen Charakters sind die Bentone nur schwer durch Wasser benetzbar.

Folgende Bentone®-Typen werden beispielsweise von der Gesellschaft Kronos Titan vertrieben: Bentone® 27, ein organisch modifiziertes Montmorillonit, Bentone® 34 (Dimethyldioctylammoniumbentonit), das nach US 2,531,427 hergestellt wird und wegen seiner lipophilen Gruppen besser im lipophilen Medium als in Wasser quillt, Bentone® 38, ein organisch modifiziertes Montmorillonit, ein cremefarbenes bis weißes Pulver, Bentone® LT, ein gereinigtes Tonmineral, Bentone® Gel MIO, ein organisch modifiziertes Montmorillonit, das in Mineralöl (SUS-71) feinst suspendiert angeboten wird (10 % Bentonit, 86,7 % Mineralöl und 3,3 % Netzmittel), Bentone® Gel IPM, ein organisch modifiziertes Bentonit, das in Isopropylmyristat suspendiert ist (10 % Bentonit, 86,7 % Isopropylmyristat, 3,3 % Netzmittel), Bentone® Gel CAO, ein organisch modifiziertes Montmorillonit, das in Ricinusöl aufgenommen ist (10 % Bentonit, 86,7 % Ricinusöl und 3,3 % Netzmittel), Bentone® Gel Lantrol, ein organisch modifiziertes Montmorillonit, das in Pastenform zur Weiterverarbeitung, insbesondere zur Herstellung kosmetischer Mittel bestimmt ist; 10 % Bentonit, 64,9 Lantrol (Wollwachsöl), 22,0 Isopropylmyristat, 3,0 Netzmittel und 0,1 p-Hydroxybenzoesäurepropylester, Bentone® Gel Lan I, eine 10 %ige Bentone® 27-Paste in einer Mischung aus Wollwachs USP und Isopropylpalmitat, Bentone® Gel Lan II, eine Bentonit-Paste in reinem, flüssigem Wollwachs, Bentone® Gel NV, eine 15 %ige Bentone® 27-Paste in Dibutylphthalat, Bentone® Gel OMS, eine Bentonit-Paste in Shellsol T. Bentone® Gel OMS 25, eine Bentonit Paste in Isoparaffinischen Kohlenwasserstoffen (Idopar® H), Bentone® Gel IPP, eine Bentonit-Paste in Isopropylpalmitat.

Die Gesamtmenge an einem oder mehreren der als Stabilisatoren bevorzugt verwendeten Hydrokolloiden wird in den fertigen kosmetischen oder dermatologischen Zubereitungen vorteilhaft kleiner als 5 Gew.-%, bevorzugt zwischen 0,05 und 3,0 Gew.-%, besonders bevorzugt zwischen 0,1 und 1,0 Gew.-%, bezogen auf das Gesamtgewicht der Zubereitungen, gewählt.

Als Komponente e) enthalten die erfindungsgemäßen Zubereitungen ein aminosubstituiertes Hydroxybenzophenon der Formel I.

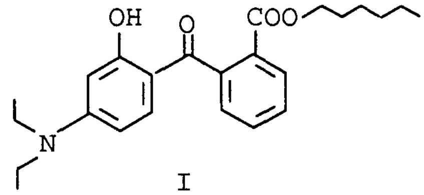

I

Diese Stoffklasse ist u.a. beschrieben in DE-A-199 17 906. Verbindung I zeichnet sich dabei besonders durch seine gute öllöslichkeit, seine gute Verarbeitbarkeit in kosmetische Zubereitungen und durch ein gutes Hautgefühl aus.

Die Menge an eingesetzter Verbindung der Formel I in den fertigen kosmetischen oder dermatologischen Zubereitungen wird vorteilhaft aus dem Bereich 0,01 Gew.-% bis 20 Gew.-%, bevorzugt von 0,1 bis 10 Gew.-%, besonders bevorzugt von 1 bis 7 Gew.-% gewählt, bezogen auf das Gesamtgewicht der Zubereitungen.

Als Grundbestandteile der erfindungsgemäßen Zubereitungen können verwendet werden:

- Wasser oder wässrige Lösungen,
- wässrige ethanolische Lösungen,
- natürliche Öle und/oder chemisch modifizierte natürliche Öle und/oder synthetische Öle,
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren,
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, dass die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Erfindung werden Lipide angesehen, deren Grenzflächenspannung gegen Wasser weniger als 30 mN/m beträgt.

Polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr, sofern die im Hauptanspruch geforderte Bedingung eingehalten wird.

Weitere polare Ölkomponenten können gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase der erfindungsgemäßen Zubereitungen einen Gehalt an $C_{12}$-$C_{15}$-Alkylbenzoat aufweist oder vollständig aus diesem besteht.

Ferner kann die Ölphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung

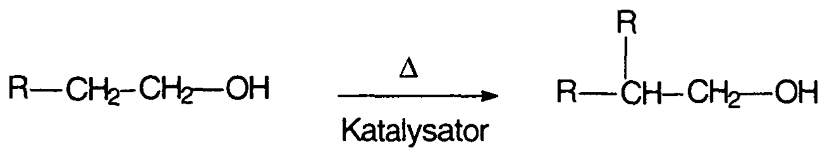


durch Oxidation eines Alkohols zu einem Aldehyd, durch AldolKondensation des Aldehyds, Abspaltung von Wasser aus dem Aldolund Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur

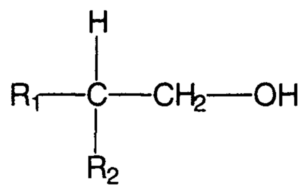

aus. Dabei bedeuten $R_1$ und $R_2$ in der Regel unverzweigte Alkylreste.

Erfindungsgemäß vorteilhaft werden der oder die Guerbet-Alkohole gewählt aus der Gruppe, bei denen

$R_1$ Propyl, Butyl, Pentyl, Hexyl, Heptyl oder Octyl und

$R_2$ Hexyl, Heptyl, Octyl, Nonyl, Decyl, Undecyl, Dodecyl, Tridecyl oder Tetradecyl bedeuten.

Erfindungsgemäß bevorzugte Guerbet-Alkohole sind das 2-Butyloctanol mit folgender chemischer Struktur

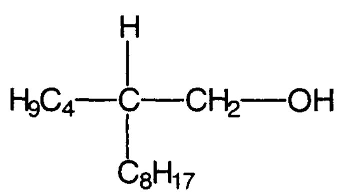

das beispielsweise unter der Handelsbezeichnung Isofol® 12 von der Gesellschaft Condea Chemie GmbH erhältlich ist sowie das 2-Hexyldecanol mit folgender chemischer Struktur

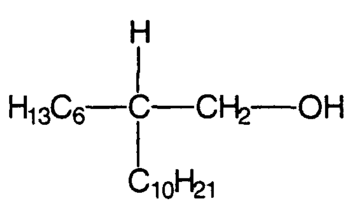

das beispielsweise unter der Handelsbezeichnung Isofol® 16 von der Gesellschaft Condea Chemie GmbH erhältlich.

Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol® 14 von der Gesellschaft Condea Chemie GmbH erhältlich.

Die Gesamtmenge an Guerbet-Alkoholen in den fertigen kosmetischen oder dermatologischen Zubereitungen wird vorteilhaft aus dem Bereich bis 25,0 Gew.-%, bevorzugt 0,5 bis 15,0 Gew.-% gewählt, bezogen auf das Gesamtgewicht der Zubereitungen.

Auch beliebige Abmischungen solcher Öl- und Wachskomponenten sind vorteilhaft im Sinne der vorliegenden

Erfindung einzusetzen. Es kann gegebenenfalls auch vorteilhaft sein, Wachse, beispielsweise Cetylpalmitat, als alleinige Lipidkomponente der Ölphase einzusetzen.

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Fett- und/oder Wachskomponenten sind chemisch modifizierte Wachse und synthetische Wachse, wie beispielsweise die unter den Handelsbezeichnungen Syncrowax HRC (Glyceryltribehenat) und Syncrowax AW 1C ($C_{18}$-$C_{36}$ -Fettsäure) bei der CRODA GmbH erhältlichen sowie Montanesterwachse, Sasolwachse, hydrierte Jojobawachse, synthetische oder modifizierte Bienenwachse (z.B. Dimethicon Copolyol Bienenwachs und/oder $C_{30-50}$ -Alkyl Bienenwachs), Polyalkylenwachse, Polyethylenglykolwachse, aber auch chemisch modifizierte Fette, wie z.B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise $C_{20}$-$C_{40}$-Alkylstearat, $C_{20}$-$C_{40}$-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan sofern die im Hauptanspruch geforderten Bedingungen eingehalten werden.

Erfindungsgemäß können die Fett- und/oder Wachskomponenten sowohl einzeln als auch im Gemisch vorliegen.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12}$-$C_{15}$-Alkylbenzoat, Capryl-Caprinsäure-triglycerid, Dicaprylylether sofern die im Hauptanspruch geforderten Bedingungen eingehalten werden.

Besonders vorteilhaft sind Mischungen aus Octyldodecanol, Capryl-Caprinsäure-triglycerid, Dicaprylylether oder Mischungen aus $C_{12}$-$C_{15}$-Alkybenzoat und 2-Ethylhexylisostearat, Mischungen aus $C_{12}$-$C_{15}$-Alkybenzoat und Isotridecylisononanoat sowie Mischungen aus $C_{12}$-$C_{15}$-Alkybenzoat, 2-Ethylhexylisostearat und Isotridecylisononanoat sofern die im Hauptanspruch geforderten Bedingungen eingehalten werden.

Die nachfolgende Tabelle 1 führt Lipide auf, die als Einzelsubstanzen oder auch im Gemisch untereinander erfindungsgemäß vorteilhaft sind. Die betreffenden Grenzflächenspannungen gegen Wasser sind in der letzten Spalte angegeben. Es ist jedoch auch vorteilhaft, Gemische aus höher- und niederpolaren und dergleichen zu verwenden.

Tabelle 1

| Handelsname | INCI-Bezeichnung | (m/Nm) |
|---|---|---|
| Isofol® 14 T | Butyl Decanol + Hexyl Decanol + Hexyl Octanol + Butyl Octanol | 27,6 |
| Isofol® 16 | Hexyl Decanol | 24,3 |
| Eutanol® G | Octyldodecanol | 24,8 |
| Cetiol® OE | Dicaprylyl Ether | 22,1 |
| Miglyol® 812 | Caprylic/Capric Triglyceride | 21,3 |
| Cegesoft® C24 | Octyl Palmitate | 23,1 |
| Isopropylstearat | Isopropyl Stearate | 21,9 |
| Estol® 1540 EHC | Octyl Octanoate | 30,0 |
| Finsolv® TN | $C_{12}$-$C_{15}$ Alkyl Benzoate | 21,8 |
| Cetiol® SN | Cetearyl Isonoanoate | 28,6 |
| Dermofeel® BGC | Butylene Glycol Dicaprylate/Dicaprate | 21,5 |
| Trivent® OCG | Tricaprylin | 20,2 |

Tabelle 1 (fortgesetzt)

| Handelsname | INCI-Bezeichnung | (m/Nm) |
|---|---|---|
| MOD | Octyldodeceyl Myristate | 22,1 |
| Cosmacol® ETI | Di-$C_{12-13}$ Alkyl Tartrate | 29,4 |
| Miglyol® 829 | Caprylic/Capric Diglyceryl Succinate | 29,5 |
| Prisorine® 2036 | Octyl Isostearate | 29,7 |
| Tegosoft® SH | Stearyl Heptanoate | 28,7 |
| Abil® Wax 9840 | Cetyl Dimethicone | 25,1 |
| Cetiol® LC | Coco-Caprylate/Caprate | 24,8 |
| IPP | Isopropyl Palmitate | 22,5 |
| Luvitol® EHO | Cetearyl Octanoate | 28,6 |
| Cetiol® 868 | Octyl Stearate | 28,4 |

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

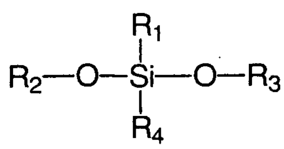

Als erfindungsgemäß vorteilhaft einzusetzenden linearen Silicone mit mehreren Siloxyleinheiten werden im allgemeinen durch Strukturelemente charakterisiert wie folgt:

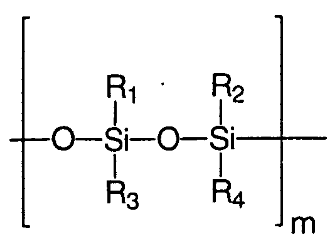
,

wobei die Siliciumatome mit gleichen oder unterschiedlichen Alkylresten und/oder Arylresten substituiert werden können, welche hier verallgemeinernd durch die Reste $R_1$ bis $R_4$ dargestellt sind, wobei die Anzahl der unterschiedlichen Reste nicht notwendig auf bis zu 4 beschränkt ist. m kann dabei Werte von 2 bis 200.000 annehmen.

Erfindungsgemäß vorteilhaft einzusetzende cyclische Silicone werden im allgemeinen durch Strukturelemente charakterisiert, wie folgt

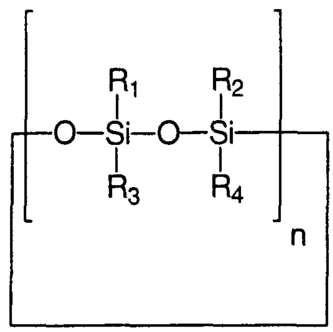

wobei die Siliciumatome mit gleichen oder unterschiedlichen Alkylresten und/oder Arylresten substituiert werden können, welche hier verallgemeinernd durch die Reste $R_1$ bis $R_4$ dargestellt sind, wobei die Anzahl der unterschiedlichen Reste nicht notwendig auf bis zu 4 beschränkt ist. n kann dabei Werte von 3/2 bis 20 annehmen. Gebrochene Werte für n berücksichtigen, dass ungeradzahlige Anzahlen von Siloxylgruppen im Cyclus vorhanden sein können.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol, das (Cetyl-Dimethicon-Copolyol (und) Polyglyceryl-4-Isostearat (und) Hexyllaurat)

Sofern denn ein geringer Anteil an Emulgatoren eingesetzt werden soll, können W/O-Emulgatoren und/oder O/W-Emulgatoren eingesetzt werden, beispielsweise vorteilhaft gewählt aus der Gruppe der folgenden Substanzen, die in der Regel als W/O-Emulgatoren wirken:

Lecithin, Lanolin, mikrokristallines Wachs (Cera microcristallina) im Gemisch mit Paraffinöl (Paraffinum liquidum), Ozokerit, hydriertem Ricinusöl, Polyglyceryl-3-Oleat, Wollwachssäuregemische, Wollwachsalkoholgemische, Pentaerythrithylisostearat, Polyglyceryl-3-Diisostearat, Bienenwachs (Cera alba) und Stearinsäure, Natriumdihydroxycetylphosphat im Gemisch mit Isopropylhydroxycetylether, Methylglucosedioleat, Methylglucosedioleat im Gemisch mit Hydroxystearat und Bienenwachs, Mineralöl im Gemisch mit Petrolatum und Ozokerit und Glyceryloleat und Lanolinalkohol, Petrolatum im Gemisch mit Ozokerit und hydriertem Ricinusöl und Glycerylisostearat und Polyglyceryl-3-oleat, PEG-7-hydriertes Rizinusöl, Ozokerit und hydriertem Ricinusöl, Polyglyceryl-4-isostearat, Polyglyceryl-4-isostearat im Gemisch mit Cetyldimethiconcopolyol und Hexyllaurat, Laurylmethiconcopolyol, Cetyldimethiconcopolyol, Acrylat/$C_{10}$-$C_{30}$-Alkylacrylat-Crosspolymer, Poloxamer 101, Polyglyceryl-2-dipolyhydroxystearat, Polyglyceryl-3-Diisostearat, Polyglyceryl-4-dipolyhydroxystearat, PEG-30-dipolyhydroxystearat, Diisostearoylpolyglyceryl-3-diisostearat, Polyglyceryl-2-dipolyhydroxystearat, Polyglyceryl-3-dipolyhydroxystearat, Polyglyceryl-4-dipolyhydroxystearat, Polyglyceryl-3-dioleat.

Es können gewünschtenfalls ein oder mehrere Emulgatoren eingesetzt werden, gewählt aus der Gruppe der folgenden Substanzen, die in der Regel als O/W-Emulgatoren wirken:

Glycerylstearat im Gemisch mit Ceteareth-20, Ceteareth-25, Ceteareth-6 im Gemisch mit Stearylalkohol, Cetylstearylalkohol im Gemisch mit PEG-40-Ricinusöl und Natriumcetylstearylsulfat, Triceteareth-4 Phosphat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl, PEG-6 Caprylsäure/Caprinsäureglyceride, Glyceryloleat im Gemisch mit Propylenglycol, Ceteth-2, Ceteth-20, Polysorbat 60, Glycerylstearat im Gemisch mit PEG-100 Stearat, Laureth-4, Ceteareth-3, Isostearylglycerylether, Cetylstearylalkohol im Gemisch mit Natrium Cetylstearylsulfat, Laureth-23, Steareth-2, Glycerylstearat im Gemisch mit PEG-30 Stearat, PEG-40-Stearat, Glycol Distearat, PEG-22-Dodecyl Glycol Copolymer, Polyglyceryl-2-PEG-4-Stearat, Ceteareth-20, Methylglucosesesquistearat, Steareth-10, PEG-20-Stearat, Steareth-2 im Gemisch mit PEG-8 Distearat, Steareth-21, Steareth-20, Isosteareth-20, PEG-45/Dodecylglycol-Copolymer, Methoxy-PEG-22/Dodecylglycol-Copolymer, PEG-20-Glycerylstearat, PEG-20-Glycerylstearat, PEG-8-Bienenwachs, Polyglyceryl-2-laurat, Isostearyldiglycerylsuccinat, Stearamidopropyl-PG-dimoniumchloridphosphat, Glycerylstearat SE, Ceteth-20, Triethylcitrat, PEG-20-Methylglucosesesquistearat, Ceteareth-12, Glycerylstearatcitrat, Cetylphosphat, Triceteareth-4-Phosphat, Trilaureth-4-Phosphat, Polyglycerylmethylglucosedistearat, Kaliumcetylphosphat, Isosteareth-10, Polyglyceryl-2-sesquiisostearat, Ceteth-10, Oleth-20, Isoceteth-20, Glycerylstearat

im Gemisch mit Ceteareth-20, Ceteareth-12, Cetylstearylalkohol und Cetylpalmitat, Cetylstearylalkohol im Gemisch mit PEG-20 Stearat, PEG-30-Stearat, PEG-40-Stearat, PEG-100-Stearat.

Es kann auch vorteilhaft im Sinne der vorliegenden Erfindung sein, insbesondere dann, wenn die Ölphase der Zubereitungen wenigstens teilweise aus Siliconölen besteht, Siliconemulgatoren zu verwenden. Die Siliconemulgatoren können vorteilhaft aus der Gruppe grenzflächenaktive Substanzen aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur:

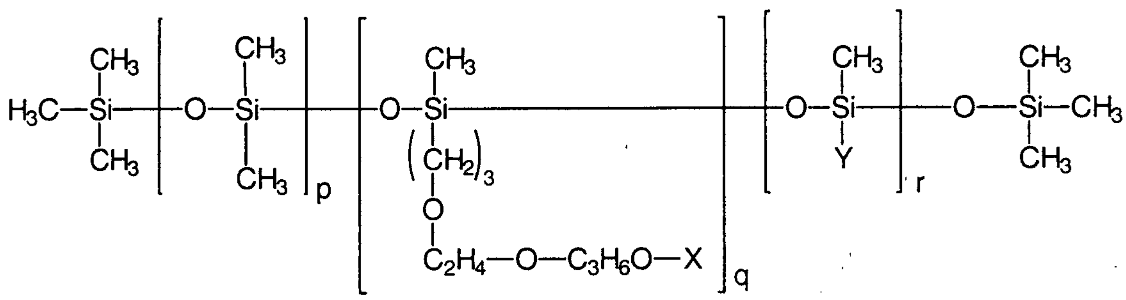

bei welcher X und Y unabhängig voneinander gewählt werden aus der Gruppe H sowie der verzweigten und unverzweigten Alkylgruppen, Acylgruppen und Alkoxygruppen mit 1 bis 24 Kohlenstoffatomen, p eine Zahl von 0 bis 200 darstellt, q eine Zahl von 1 bis 40 darstellt, und r eine Zahl von 1 bis 100 darstellt.

Ein Beispiel für besonders vorteilhaft im Sinne der vorliegenden Erfindung zu verwendende Silikonemulgatoren sind Dimethiconcopolyole, welche von der Gesellschaft Th. Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden.

Ein weiteres Beispiel für besonders vorteilhaft im Sinne der vorliegenden Erfindung zu verwendende grenzflächenaktiven Substanzen ist das Cetyl Dimethiconcopolyol, welches von der Gesellschaft Th. Goldschmidt AG unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von der Gesellschaft Th. Goldschmidt AG unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Sofern denn ein geringer Anteil an Siliconemulgatoren eingesetzt werden soll, liegt deren Gesamtmenge in den erfindungsgemäßen kosmetischen oder dermatologischen Zubereitungen vorteilhafterweise im Bereich von 0,1 bis 2,0 Gew.-%, bevorzugt 0,5 bis 1,5 Gew.-%, bezogen auf das Gesamtgewicht der Zubereitungen.

Erfindungsgemäße Zubereitungen im Sinne der vorliegenden Erfindung, z.B. in Form einer Hautschutzcreme, einer Hautlotion, einer kosmetischen Milch, beispielsweise in Form einer Sonnenschutzcreme oder einer Sonnenschutzmilch, sind vorteilhaft und enthalten z.B. Fette, Öle, Wachse und/oder andere Fettkörper, sowie Wasser und gewünschtenfalls einen oder mehrere Emulgatoren, wie sie üblicherweise für einen solchen Typ der Formulierung verwendet werden.

Ebenso wie Zubereitungen von flüssiger und fester Konsistenz als kosmetische Reinigungslotionen oder Reinigungscremes Verwendung finden, können auch die erfindungsgemäßen Zubereitungen versprühbare Reinigungszubereitungen ("Reinigungssprays") darstellen, welche beispielsweise zum Entfernen von Schminken und/oder Make-up oder als milde Waschlotion - ggf. auch für unreine Haut - verwendet werden. Derartige Reinigungszubereitungen können vorteilhaft ferner als sogenannte "rinse-off-Präparate" angewendet werden, welche nach der Anwendung von der Haut abgespült werden.

Es ist dem Fachmann natürlich bekannt, dass anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch,

Sonnenschutzlotion, Nährcreme, Tagesoder Nachtcreme usw. Es ist gegebenenfalls möglich und vorteilhaft, die erfindungsgemäßen Zusammensetzungen als Grundlage für pharmazeutische Formulierungen zu verwenden.

Es ist ebenfalls von Vorteil, die erfindungsgemäßen Zubereitungen für dekorative Kosmetika (Make-Up-Formulierungen) zu verwenden.

Günstig sind auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens einen Breitbandfilter und/oder mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescremes gewöhnlich UV-A- bzw. UV-B-Filtersubstanzen eingearbeitet.

Vorteilhafte Breitbandfilter, W-A- oder UV-B-Filtersubstanzen sind beispielsweise Bis-Resorcinyltriazinderivate mit der folgenden Struktur:

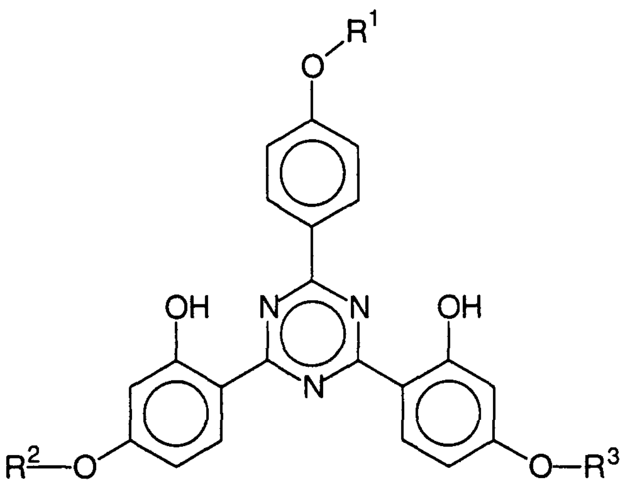

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethylhexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Auch andere UV-Filtersubstanzen, welche das Strukturmotiv

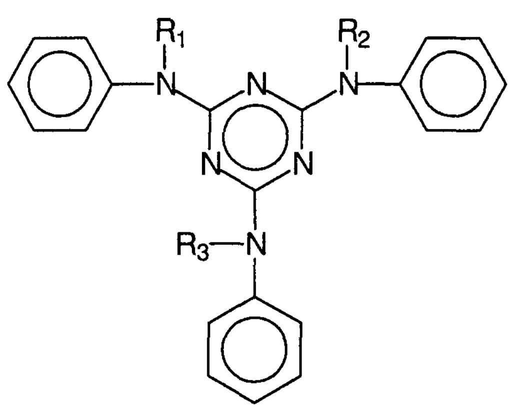

aufweisen, sind vorteilhafte UV-Filtersubstanzen im Sinne der vorliegenden Erfindung, beispielsweise die in der Europäischen Offenlegungsschrift EP 570 838 Al beschriebenen s-Triazinderivate, deren chemische Struktur durch die generische Formel

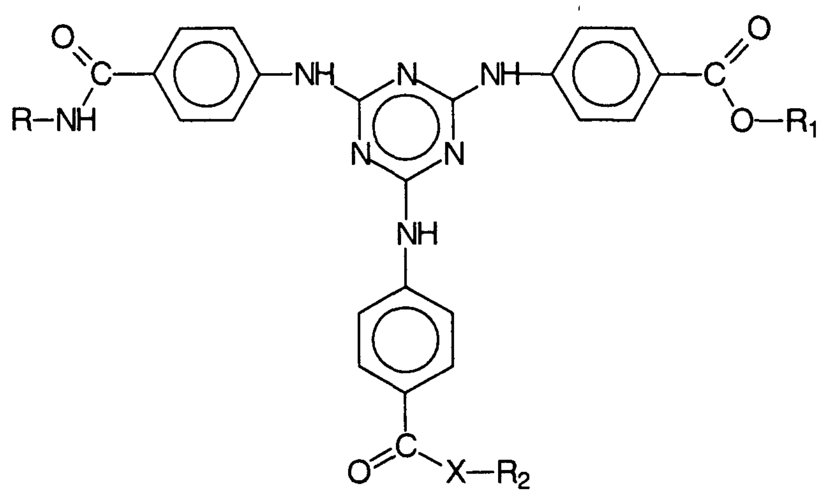

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

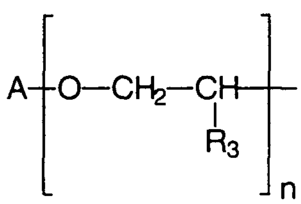

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

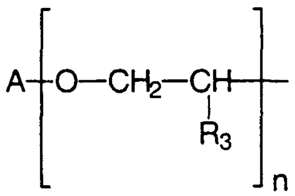

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel

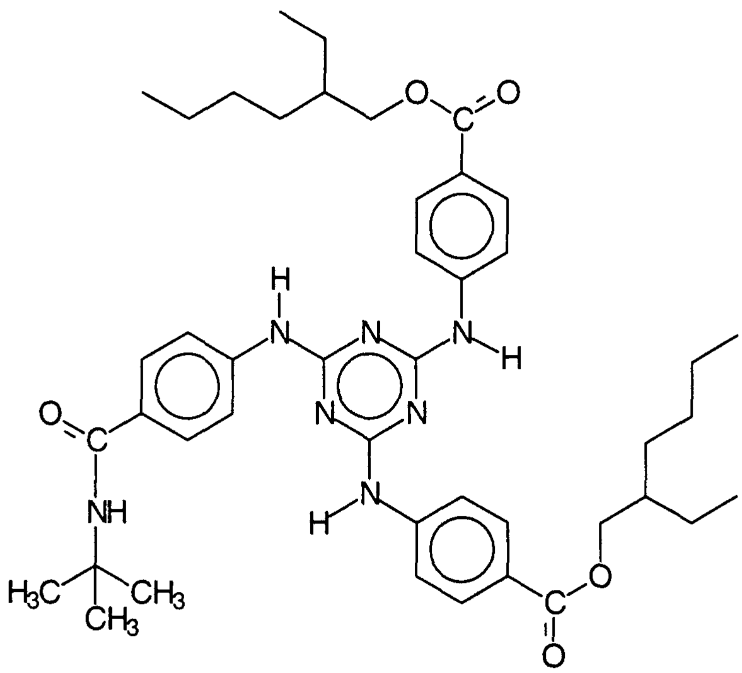

wiedergegeben wird, welches im Folgenden auch als Dioctylbutylamidotriazon (INCI: Diethylhexylbutamidotriazone) bezeichnet wird und unter der Handelsbezeichnung UVASORB® HEB bei Sigma 3V erhältlich ist.

Vorteilhaft im Sinne der vorliegenden Erfindung ist auch ein symmetrisch substituiertes s-Triazin, das 4,4',4''-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel

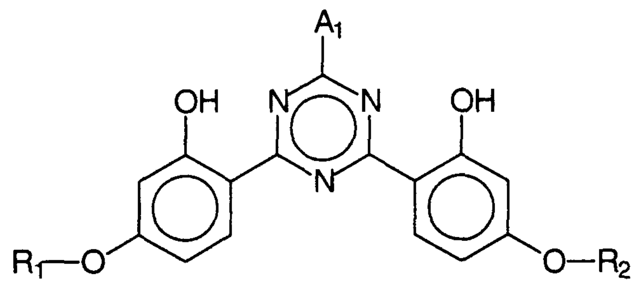

wiedergegeben wird, wobei $R_1$ und $R_2$ u.a. $C_3$-$C_{18}$-Alkyl oder $C_2$-$C_{18}$-Alkenyl und $A_1$ einen aromatischen Rest repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethylcarboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxysilylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Die gegebenenfalls eingesetzte Gesamtmenge an einem oder mehreren Triazinderivaten in den fertigen kosmetischen oder dermatologischen Zubereitungen wird vorteilhaft aus dem Bereich 0,01 Gew.-% bis 15 Gew.-%, vorzugsweise von 0,1 bis 10 Gew.-% gewählt, jeweils bezogen auf das Gesamtgewicht der Zubereitungen.

Vorteilhafte sulfonierte, wasserlösliche UV-Filter im Sinne der vorliegenden Erfindung sind:

Phenylen-1,4-bis-(2-benzimidazyl)-3,3'-5,5'-tetrasulfonsäure, welche sich durch folgende Struktur auszeichnet:

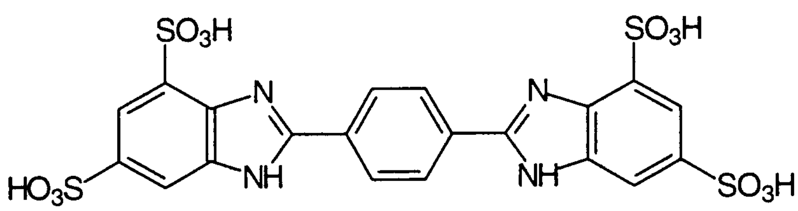

Sowie ihre Salze, besonders die entsprechenden Natrium-, Kaliumoder Triethanolammonium-Salze, insbesondere das Phenylen-1,4-bis-(2-benzimidazyl)-3,3'-5,5'-tetrasulfonsäure-bis-natriumsalz

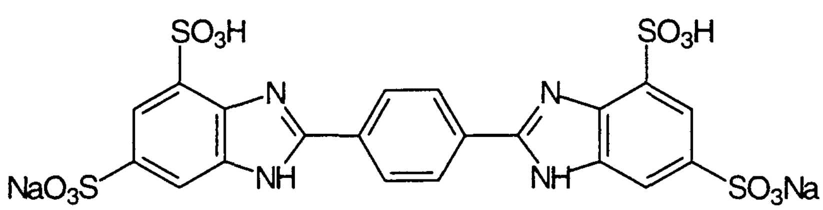

mit der INCI-Bezeichnung Bisimidazylate (CAS-Nr.: 180898-37-7), welches beispielsweise unter der Handelsbezeichnung Neo Heliopan® AP bei Haarmann & Reimer erhältlich ist.

Ein weiterer im Sinne der vorliegenden Erfindung vorteilhafter sulfonierter UV-Filter sind die Salze der 2-Phenylbenzimidazol-5-sulfonsäure, wie ihr Natrium-, Kalium- oder ihr Triethanolammonium-Salz, sowie die Sulfonsäure selbst

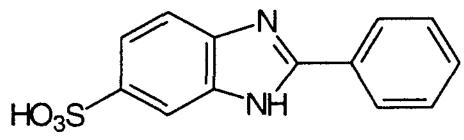

mit der INCI Bezeichnung Phenylbenzimidazole Sulfonsäure (CAS.-Nr. 27503-81-7), welches beispielsweise unter der Handelsbezeichnung Eusolex® 232 bei Merck oder unter Neo Heliopan® Hydro bei Haarmann & Reimer erhältlich ist.

Eine weiterer vorteilhafter sulfonierter UV-Filter ist die 3,3'-(1,4-Phenylendimethylene) bis (7,7-dimethyl-2-oxo-bicyclo-[2.2.1] hept-1-ylmethane Sulfonsäure, wie ihr Natrium-, Kalium- oder ihr Triethanolammonium-Salz, sowie die Sulfonsäure selbst:

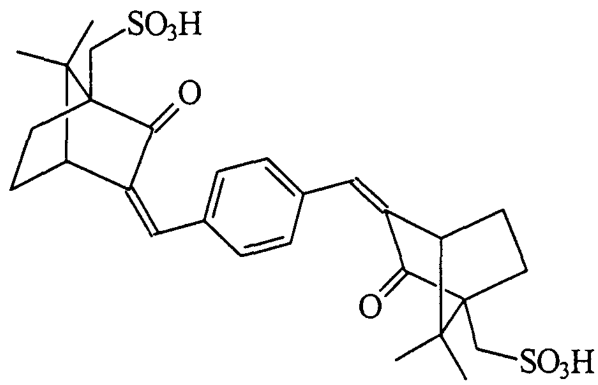

mit der INCI-Bezeichnung Terephthalidene Dicampher Sulfonsäure (CAS.-Nr.: 90457-82-2), welche beispielsweise unter dem Handelsnamen Mexoryl® SX von der Fa. Chimex erhältlich ist.

Weitere vorteilhafte wasserlösliche UV-B- und/oder Breitband-Filtersubstanzen sind z.B.:

Sulfonsäure-Derivate des 3-Benzylidencamphers, wie z.B. 4-(2-Oxo-3-bornylidenmethyl)benzolsulfonsäure, 2-Methyl-5 - (2-oxo-3-bornylidenmethyl)sulfonsäure und deren Salze.

Die gegebenenfalls eingesetzte Gesamtmenge an einer oder mehreren sulfonierten UV-Filtersubstanzen in den fertigen kosmetischen oder dermatologischen Zubereitungen wird vorteilhaft aus dem Bereich 0,01 Gew.-% bis 20 Gew.-%, vorzugsweise von 0,1 bis 10 Gew.-% gewählt, jeweils bezogen auf das Gesamtgewicht der Zubereitungen.

Die UV-B- und/oder Breitband-Filter können öllöslich oder wasserlöslich sein. Vorteilhafte öllösliche UV-B- und/oder Breitband-Filtersubstanzen sind z,B.:

3-Benzylidencampher-Derivate, vorzugsweise 3-(4-Methylbenzyliden)campher, 3-Benzylidencampher;

4-Aminobenzoesäure-Derivate, vorzugsweise 4-(Dimethylamino)-benzoesäure(2-ethylhexyl)ester, 4-(Dimethylamino)benzoesäureamylester, 4-Bis(polyethoxy)amino-benzoesäurepolyethoxyethylester (unter der Handelsbezeichnung Uvinul® P25 von der Fa. BASF erhältlich);

Derivate des Benzophenons, vorzugsweise 2-Hydroxy-4-methoxybenzophenon (unter der Handelsbezeichnung Uvinul® M40 von der Fa. BASF erhältlich), 2-Hydroxy-4-methoxy-4'-methylbenzophenon, 2,2'-Dihydroxy-4-methoxybenzophenon, 2-Hydroxy-4-methoxybenzophenon-5-sulfonsäure (unter der Handelsbezeichnung Uvinul® MS40 von der Fa. BASF erhältlich);

sowie an Polymere gebundene UV-Filter.

Besonders vorteilhafte bei Raumtemperatur flüssige UV-Filtersubstanzen im Sinne der vorliegenden Erfindung sind Homomenthylsalicylat, 2-Ethylhexyl-2-cyano-3,3-diphenylacrylat, 2-Ethylhexyl-2-hydroxybenzoat und Ester der Zimtsäure, vorzugsweise 4-Methoxyzimtsäure(2-ethylhexyl)ester und 4-Methoxyzimtsäureisopentylester.

Homomenthylsalicylat (INCI: Homosalate) zeichnet sich durch die folgende Struktur aus:

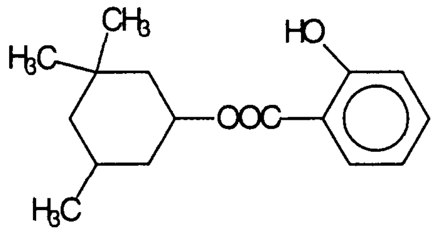

2-Ethylhexyl-2-cyano-3,3-diphenylacrylat (INCI: Octocrylene) ist von BASF unter der Bezeichnung Uvinul® N 539T erhältlich und zeichnet sich durch folgende Struktur aus:

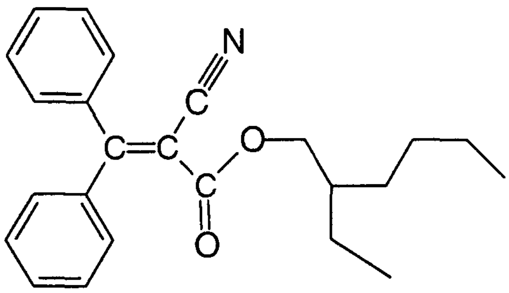

2-Ethylhexyl-2-hydroxybenzoat (2-Ethylhexylsalicylat, Octylsalicylat, INCI: Ethylhexyl Salicylate) ist beispielsweise bei Haarmann & Reimer unter der Handelsbezeichnung Neo Heliopan® OS erhältlich und zeichnet sich durch die folgende Struktur aus:

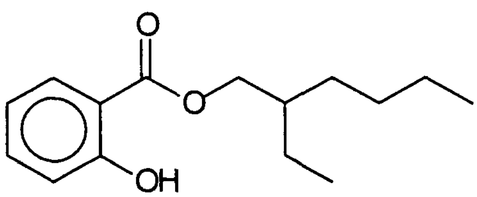

4-Methoxyzimtsäure(2-ethylhexyl)ester (2-Ethylhexyl-4-methoxycinnamat, INCI: Ethylhexyl Methoxycinnamate) ist beispielsweise bei Fa. BASF unter der Handelsbezeichnung Uvinul® MC 80 erhältlich und zeichnet sich durch die folgende Struktur aus:

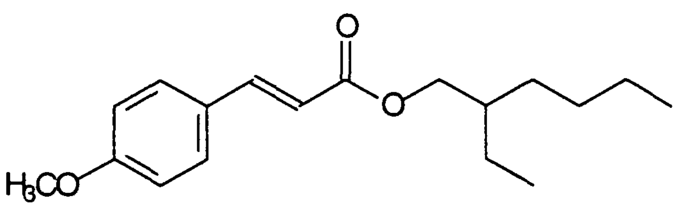

4-Methoxyzimtsäureisopentylester (Isopentyl-4-methoxycinnamat, INCI: Isoamyl p-Methoxycinnamate) ist beispielsweise bei Haarmann & Reimer unter der Handelsbezeichnung Neo Heliopan® E 1000 erhältlich und zeichnet sich durch die folgende Struktur aus:

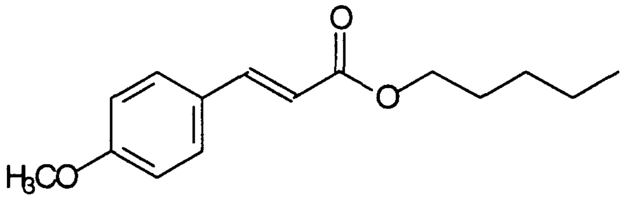

Eine weitere vorteilhafte, bei Raumtemperatur flüssige UV-Filter Substanz im Sinne der vorliegenden Erfindung (3-(4-(2,2-bis-Ethoxycarbonylvinyl)-phenoxy)propenyl)-methylsiloxan/Dimethylsiloxan Copolymer, welches beispielsweise bei Hoffmann-La Roche unter der Handelsbezeichnung Parsol® SLX erhältlich ist.

Die gegebenenfalls eingesetzte Gesamtmenge an einer oder mehreren bei Raumtemperatur flüssigen UV-Filtersubstanzen in den fertigen kosmetischen oder dermatologischen Zubereitungen wird vorteilhaft aus dem Bereich 0,1 Gew.-% bis 30 Gew.-%, vorzugsweise von 0,5 bis 20 Gew.-% gewählt, jeweils bezogen auf das Gesamtgewicht der Zubereitungen.

Vorteilhafte Dibenzoylmethanderivate im Sinne der vorliegenden Erfindung sind, insbesondere das 4-(tert. -Butyl)-4'-methoxydibenzoyimethan (CAS-Nr. 70356-09-1), welches von BASF unter der Marke Uvinul® BMBM und von Merck unter der Handelsbezeichnung Eusolex® 9020 verkauft wird zeichnet sich durch folgende Struktur aus:

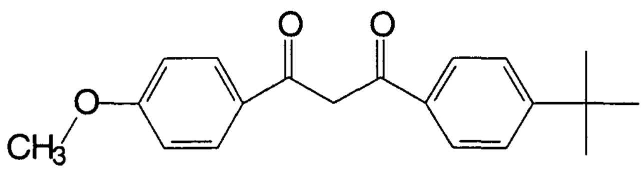

Ein weiters vorteilhaftes Dibenzoylmethanderivat ist das 4-Isopropyl-Dibenzoylmethan (CAS-Nr. 63250-25-9), welches von Merck unter dem Namen Eusolex® 8020 verkauft wird. Das Eusolex 8020 zeichnet sich durch folgende Struktur aus:

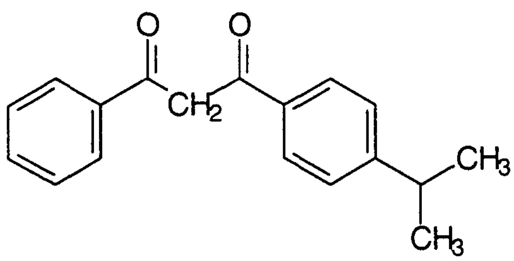

Benzotriazole zeichnen sich durch die folgende Strukturformel aus:

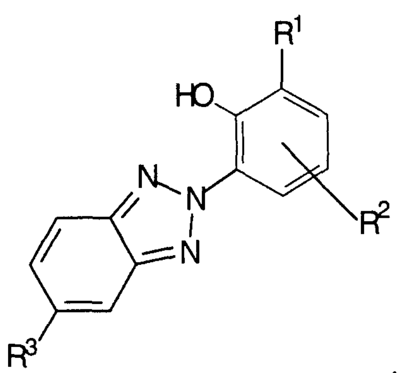

,

worin
$R^1$ und $R^2$ unabhängig voneinander lineare oder verzweigte, gesättigte oder ungesättigte, substituierte (z.B. mit einem Phenylrest substituierte) oder unsubstituierte Alkylreste mit 1 bis 18 Kohlenstoffatomen und/oder Polymerreste, welche selbst nicht UV-Strahlen absorbieren (wie z.B. Silikonreste, Acrylatreste und dergleichen mehr), darstellen können und $R_3$ aus der Gruppe H oder Alkylrest mit 1 bis 18 Kohlenstoffatomen gewählt wird.

Ein vorteilhaftes Benzotriazol im Sinne der vorliegenden Erfindung ist das 2,2'-Methylen-bis-(6-(2H-benzotriazol-2-yl)-4-(1,1,3,3-tetramethylbutyl)-phenol), ein Breitbandfilter, welcher durch die chemische Strukturformel

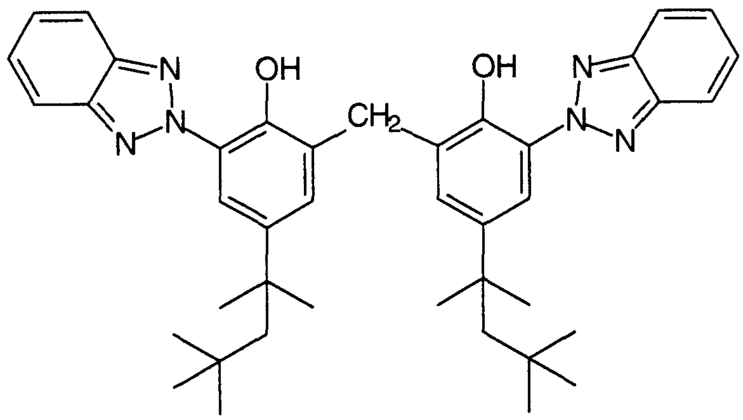

gekennzeichnet ist und unter der Handelsbezeichnung Tinosorb® M bei der CIBA-Chemikalien GmbH erhältlich ist.

Vorteilhaftes Benzotriazol im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]-propyl]-phenol (CAS-Nr.: 155633-54-8) mit der INCI-Bezeichnung Drometrizole Trisiloxane, welches von Fa. Chimex unter der Marke Mexoryl® XL verkauft wird und durch die folgende chemische Strukturformel

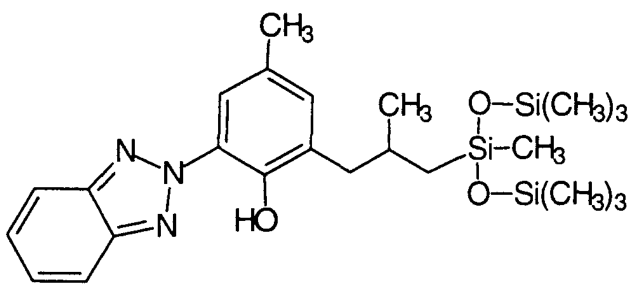

gekennzeichnet ist.

Weitere vorteilhafte Benzotriazole im Sinne der vorliegenden Erfindung sind [2,4'-Dihydroxy-3-(2H-benzotriazol-2-yl)-5-(1,1,3,3-tetramethylbutyl)-2'-n-octoxy-5'-benzoyl]diphenylmethan, 2,2' Methylen-bis-[6-(2H-benzotriazol-2-yl)-4-(methyl)phenol], 2,2'-Methylen-bis-[6-(2H-benzotriazol-2-yl)-4-(1,1,3,3-tetramethylbutyl)phenol], 2-(2'-Hydroxy-5'-octylphenyl)-benzotriazol, 2-(2'-Hydroxy-3',5'-di-t-amylphenyl)benzotriazol und 2-(2'-Hydroxy-5'-methylphenyl) benzotriazol.

Die gegebenenfalls eingesetzte Gesamtmenge an einem oder mehreren Benzotriazolen in den fertigen kosmetischen oder dermatologischen Zubereitungen wird vorteilhaft aus dem Bereich 0,1 bis 20 Gew.-%, vorzugsweise von 0,5 bis 15 Gew.-%, ganz besonders bevorzugt von 0,5 bis 10 Gew.-% gewählt, jeweils bezogen auf das Gesamtgewicht der Zubereitungen.

Ein weiterer im Sinne der vorliegenden Erfindung vorteilhafter UV-A-Filter ist der in EP-A-0 895 776 beschriebene 2-(4-Ethoxyanilinomethylen)-propandicarbonsäure-diethylester der folgenden Formel.

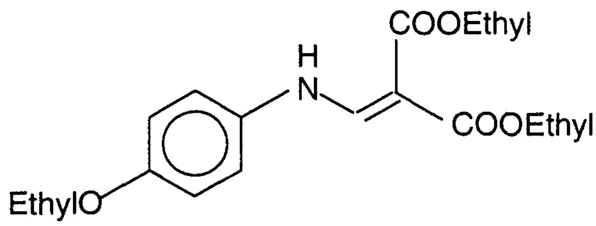

Erfindungsgemäße kosmetische und dermatologische Zubereitungen enthalten ferner vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. Fe2O3), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Diese Pigmente sind röntgenamorph oder nicht-röntgenamorph. Besonders bevorzugt handelt es sich um Pigmente auf der Basis von TiO2.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, dass ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt.

Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Die gegebenenfalls eingesetzte Gesamtmenge an einem oder mehreren röntgenamorphen Oxidpigmenten in den fertigen kosmetischen oder dermatologischen Zubereitungen wird vorteilhaft aus dem Bereich 0,1 bis 20 Gew.-%, vorzugsweise von 0,5 bis 10 Gew.-%, ganz besonders bevorzugt von 1 bis 5 Gew.-% gewählt, jeweils bezogen auf das Gesamtgewicht der Zubereitungen.

Die nicht-röntgenamorphen anorganischen Pigmente liegen erfindungsgemäß vorteilhaft in hydrophober Form vor, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden.

Eines solcher Verfahren besteht beispielsweise darin, dass die hydrophobe Oberflächenschicht nach einer Reaktion gemäß

$$n\ TiO_2 + m\ (RO)3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS/33 14 742 dargestellte hydrophobisierte Pigmente sind von Vorteil.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel), Octyltrimethoxysilan oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z-Cote® HP1 | 2 % Dimethicone | BASF |
| Z-Cote® | - | BASF |
| ZnO NDM | 5 % Dimethicone | H&R |
| MZ-505 S | 5 % Methicone | Tayca Corp. |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid/ Stearinsäure | Tayca Corporation |
| MT-100Z | Aluminiumhydroxid/ Stearinsäure | Tayca Corporation |
| Eusolex® T-2000 | Alumina/Simethicone | Merck KgaA |
| Titandioxid T805 (Uvinul® $TiO_2$) | Octyltrimethoxysilan | Degussa, BASF |

Vorteilhafte $TiO_2$-Pigmente sind beispielsweise unter der Handelsbezeichnung Uvinul® $TiO_2$, vorteilhafte $TiO_2/Fe_2O_3$-Mischoxide unter der Handelsbezeichnung Uvinul® $TiO_2$ A von der Firma BASF erhältlich.

Die gegebenenfalls verwendete Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 bis 30 Gew.-%, bevorzugt 0,1 bis 10,0, insbesondere 0,5 bis 6,0 Gew.-% gewählt, bezogen auf das Gesamtgewicht der Zubereitungen.

Zusätzlich zu den bereits beschriebenen erfindungsgemäßen Bestandteilen können die erfindungsgemäßen kosmetischen und dermatologischen Zubereitungen weitere kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, weichmachende, anfeuchtende und/oder feuchthaltende Substanzen, Fette, Öle, Wachse oder andere übliche Bestandteile einer kosmetischen oder dermatologischen Formulierung wie Schaumstabilisatoren, Elektrolyte, organische Lösungsmittel oder Silikonderivate.

In der Lebensmitteltechnologie zugelassene Konservierungsmittel, die mit ihrer E-Nummer nachfolgend aufgeführt sind, sind erfindungsgemäß vorteilhaft zu verwenden.

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |

(fortgesetzt)

| | | | |
|---|---|---|---|
| E 221 | Natriumsulfit | E 280 | Propionsäure |
| E 222 | Natriumyhydrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner sind erfindungsgemäß in der Kosmetik gebräuchliche Konservierungsmittel oder Konservierungshilfsstoffe Dibromdicyanobutan (2-Brom-2-brommethylglutarodinitril), 3-Iod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol geeignet. + Formaldehydabspalter.

Ferner sind Phenylhydroxyalkylether, insbesondere die unter der Bezeichnung Phenoxyethanol bekannte Verbindung aufgrund ihrer bakteriziden und fungiziden Wirkungen auf eine Anzahl von Mikroorganismen als Konservierungsmittel geeignet.

Auch andere keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Es ist darüberhinaus vorteilhaft, den Zubereitungen im Sinne der vorliegenden Erfindung weitere antiirritative oder antientzündliche Wirkstoffe zuzugeben, insbesondere Batylalkohol (α-Octadecylglycerylether), Selachylalkohol (α-9-Octadecenylglycerylether), Chimylalkohol (α-Hexadecylglycerylether), Bisabolol und/oder Panthenol.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Zubereitungen gemäß der vorliegenden Erfindung können auch Verwendung als Grundlage für kosmetische oder dermatologische Desodorantien bzw. Antitranspirantien finden. Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Weitere Zusatzstoffe können u.a. quaternäre Tenside sein. Diese Verbindungen enthalten mindestens ein N-Atom, das mit 4 Alkyloder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Ferner sind auch sogenannte Metallseifen (d. h. die Salze höherer Fettsäuren mit Ausnahme der Alkalisalze) vorteilhafte Ölverdickungsmittel im Sinne der vorliegenden Erfindung, wie beispielsweise Aluminium-Stearat, Zink-Stearat und/oder MagnesiumStearat.

Ebenfalls vorteilhaft ist, Zubereitungen gemäß der Erfindung amphotere bzw. zwitterionische Tenside (z.B. Cocoamidopropylbetain) und Moisturizer (z.B. Betain) zuzusetzen. Vorteilhaft zu verwendende amphotere Tenside sind beispielsweise Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat, N-Alkylaminosäuren, beispielsweise Aminopropylalkylglutamid, Alkylaminopropionsäure, Natriumalkylimidodipropionat und Lauroamphocarboxyglycinat.

Eine erstaunliche Eigenschaft der erfindungsgemäße Zubereitungen ist, dass diese sehr gute Vehikel für kosmetische oder dermatologische Wirkstoffe in die Haut sind, wobei bevorzugte Wirkstoffe die vorab erwähnten Antioxidantien sind, welche die Haut vor oxidativer Beanspruchung schützen können.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine der Bund D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die γ-Linolensäure, Ölsäure, Eicosapentaensäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit®.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wässrige bzw. organische Extrakte aus Pflanzen oder Pflanzenteilen umfasst, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z.B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben):

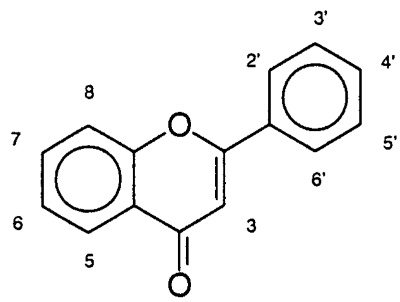

Einige der wichtigeren Flavone, welche auch bevorzugt in erfindungsgemäßen Zubereitungen eingesetzt werden können, sind in der nachstehenden Tabelle 2 aufgeführt:

Tabelle 2

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | - | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden bevorzugt die Flavonoide ausgewählt aus der Gruppe der Substanzen der generischen Strukturformel

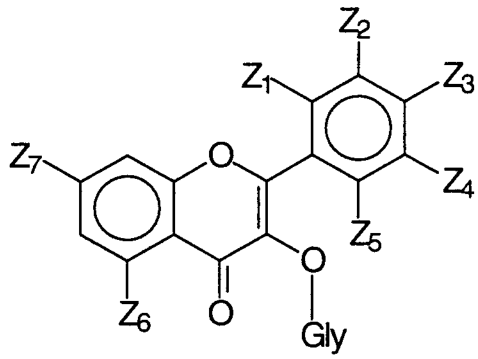

wobei $Z_1$ bis $Z_7$ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel

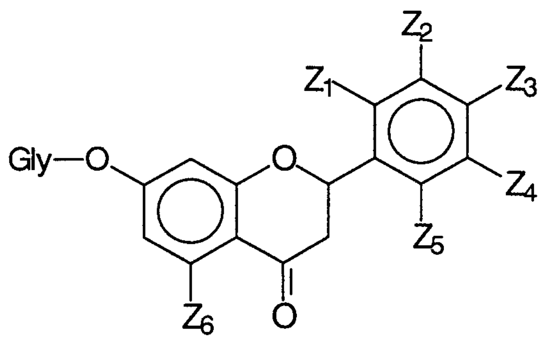

wobei $Z_1$ bis $Z_6$ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel

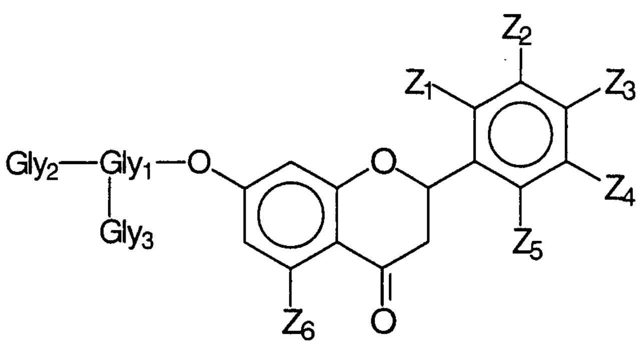

wobei $Gly_1$, $Gly_2$ und $Gly_3$ unabhängig voneinander Monoglycosidreste oder darstellen. $Gly_2$ bzw. $Gly_3$ können auch einzeln oder gemeinsam Absättigungen durch Wasserstoffatome darstellen.

Bevorzugt werden $Gly_1$, $Gly_2$ und $Gly_3$ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden $Z_1$ bis $Z_5$ unabhängig voneinander gewählt aus der Gruppe H, OH, Methoxy-, Ethoxy- sowie 2-Hydroxyethoxy-, und die Flavonglycoside haben die Struktur

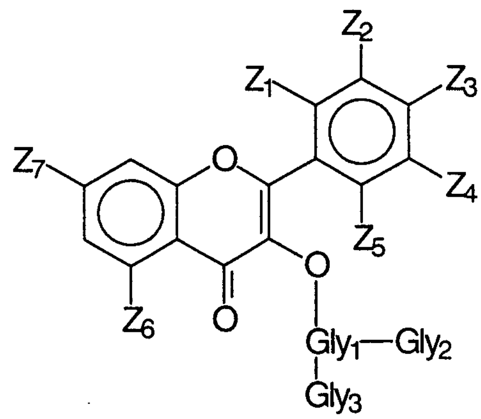

Besonders vorteilhaft werden die erfindungsgemäßen Flavonglycoside aus der Gruppe, welche durch die folgende Struktur wiedergegeben werden:

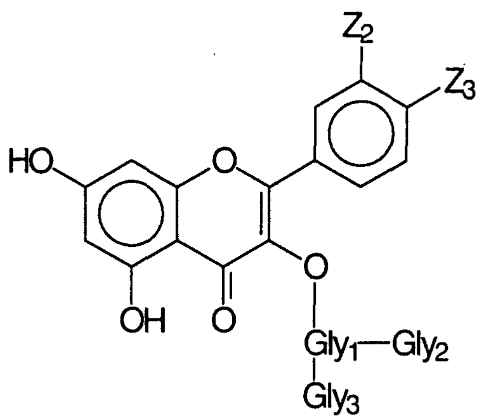

wobei $Gly_1$, $Gly_2$ und $Gly_3$ unabhängig voneinander Monoglycosidreste oder darstellen. $Gly_2$ bzw. $Gly_3$ können auch einzeln oder gemeinsam Absättigungen durch Wasserstoffatome darstellen.

Bevorzugt werden $Gly_1$, $Gly_2$ und $Gly_3$ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'methoxyflavanon-7-rutinosid, Hesperidosid, Hesperetin-7-Orutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris-(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomarein (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, dem oder die Wirkstoffe aus der Gruppe der Ubichinone und Plastochinone zu wählen.

Ubichinone zeichnen sich durch die Strukturformel

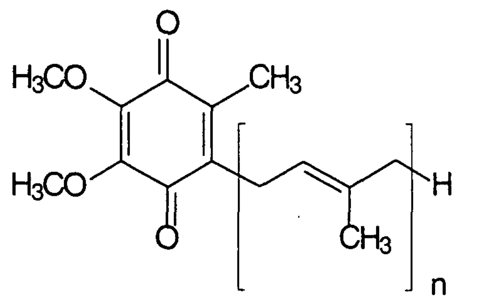

aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z.B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

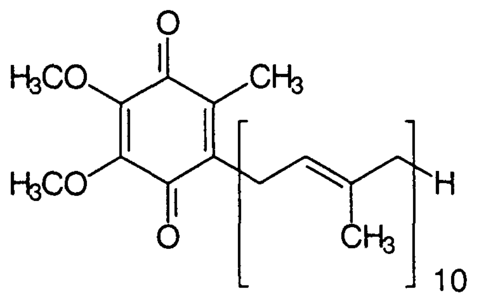

Plastochinone weisen die allgemeine Strukturformel

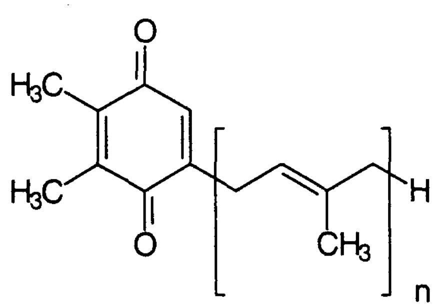

auf. Plastoschinone unterscheiden sich in der Anzahl n der Isopren-Reste und werden entsprechend bezeichnet, z.B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus:

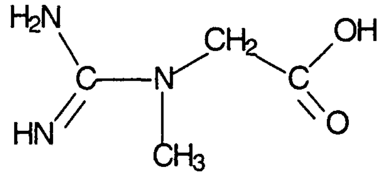

Bevorzugte Derivate sind Kreatinphosphat sowie Kreatinsulfat, Kreatinacetat, Kreatinascorbat und die an der

Carboxylgruppe mit mono- oder polyfunktionalen Alkoholen veresterten Derivate.

Ein weiterer vorteilhafter Wirkstoff ist L-Carnitin [3-Hydroxy-4-(trimethylammonio)-buttersäurebetain]. Auch Acyl-Carnitine, welche gewählt aus der Gruppe der Substanzen der folgenden allgemeinen Strukturformel

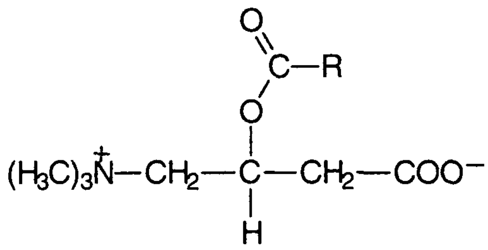

wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Sericosid, Pyridoxol, Vitamin K, Biotin und Aromastoffe.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Es kann auch gegebenenfalls vorteilhaft im Sinne der vorliegenden Erfindung sein, den Zubereitungen gemäß der Erfindung Farbstoffe und/oder Farbpigmente einzuverleiben.

Die Farbstoffe und Farbpigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z.B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxy-naphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |

(fortgesetzt)

| Chemische oder sonstige Bezeichnung | CIN | Farbe |
|---|---|---|
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |

| Chemische oder sonstige Bezeichnung | CIN | Farbe |
|---|---|---|
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxy-naphthalin | 15580 | rot |
| 1-(4',(8')-Sulfosäurenaphthylazo)-2-hydroxy-naphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthyl-carbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-l-phenylazo)-2-naphthol-6-sulfosäure | 15980 | orange |
| 1-(4-Sulfo-l-phenylazo)-2-naphthol-6-sulfosäure | 15985 | gelb |
| Allura Red | 16035 | rot |
| 1-(4-Sulfo-1-naphthylazo)-2-naphthol-3,6-disulfosaure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2",4"-dimethyl)-bis-phenylazo)-1,3-dihydroxybenzol | 20170 | orange |
| Acid Black 1 | 20470 | schwarz |

(fortgesetzt)

| Chemische oder sonstige Bezeichnung | CIN | Farbe |
|---|---|---|
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |

| Chemische oder sonstige Bezeichnung | CIN | Farbe |
|---|---|---|
| Food Yellow | 40800 | orange |
| trans-ß-Apo-8'-Carotinaldehyd ($C_{30}$) | 40820 | orange |
| trans-Apo-8'-Carotinsäure($C_{30}$)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl)-$\Delta^{2,5}$-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |

(fortgesetzt)

| Chemische oder sonstige Bezeichnung | CIN | Farbe |
|---|---|---|
| Acid Red 98 | 45405 | rot |
| 3',4',5',6'-Tetrachlor-2,4,5,7-tetrabromfluorescein | 45410 45410 | rot rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| l-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |

| Chemische oder sonstige Bezeichnung | CIN | Farbe |
|---|---|---|
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |

(fortgesetzt)

| Chemische oder sonstige Bezeichnung | CIN | Farbe |
|---|---|---|
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Manganammoniumdiphosphat | 77742 | violett |

| Chemische oder sonstige Bezeichnung | CIN | Farbe |
|---|---|---|
| Manganphosphat; $Mn_3(PO_4)_2 \cdot 7$ H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |

(fortgesetzt)

| Chemische oder sonstige Bezeichnung | CIN | Farbe |
|---|---|---|
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Ferner vorteilhaft sind öllösliche Naturfarbstoffe, wie z.B. Paprikaextrakte, β-Carotin oder Cochenille.

Vorteilhaft im Sinne der vorliegenden Erfindung sind ferner Gelcremes mit einem Gehalt an Perlglanzpigmenten. Bevorzugt sind insbesondere die im folgenden aufgelisteten Arten von Perlglanzpigmenten:

Natürliche Perlglanzpigmente, wie z.B.

"Fischsilber" (Guanin/Hypoxanthin-Mischkristalle aus Fischschuppen), "Perlmutt" (vermahlene Muschelschalen) und Monokristalline Perlglanzpigmente wie z.B. Bismuthoxychlorid (BiOCl),

Schicht-Substrat Pigmente: z.B. Glimmer/Metalloxid.

Basis für Perlglanzpigmente sind beispielsweise pulverförmige Pigmente oder Rizinusöldispersionen von Bismutoxychlorid und/oder Titandioxid sowie Bismutoxychlorid und/oder Titandioxid auf Glimmer. Insbesondere vorteilhaft ist z.B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung/Schichtdicke | Farbe |
|---|---|---|
| Silberweiße Perlglanzpigmente | $TiO_2$: 40 - 60 nm | silber |
| Interferenzpigmente | $TiO_2$: 60 - 80 nm | gelb |
| | $TiO_2$: 80 - 100 nm | rot |
| | $TiO_2$: 100 - 140 nm | blau |
| | $TiO_2$: 120 - 160 nm | grün |
| Farbglanzpigmente | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| Kombinationspigmente | $TiO_2$ / $Fe_2O_3$ | Goldtöne |
| | $TiO_2$ / $Cr_2O_3$ | grün |
| | $TiO_2$ / Berliner Blau | tiefblau |
| | $TiO_2$ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z.B. Silica und dergleichen mehr. Vorteilhaft sind z.B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von $SiO_2$ hergestellt werden. Solche Pigmente, die auch zusätzlich gonichromatische Effekte haben können, sind z.B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40 nm bis 180 μm zusätzlich zu der Farbe einen Glitzereffekt auf.

Besonders vorteilhaft sind ferner auch Effektpigmente, welche unter der Handelsbezeichnung Metasomes Standard/Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z.B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Die Herstellung erfindungsgemäßer Zubereitungen erfolgt unter den dem Fachmanne geläufigen Bedingungen. Es werden in der Regel die Bestandteile der Ölphase bzw. der Wasserphase gesondert zusammengegeben und erwärmt, und sodann unter Rühren und, besonders vorteilhaft, unter Homogenisieren, ganz besonders vorteilhaft unter Rühren mit mittlerem bis hohen Energieeintrag, vorteilhaft mit Hilfe einer Zahnkranzdispergiermaschine mit einer Umdrehungszahl bis maximal 10000 U/min, vorzugsweise von 2500 bis 7700 U/min, miteinander vereinigt.

Anhand der nachfolgenden Beispiele (Formulier-Rezepturen) soll die vorliegende Erfindung näher erläutert werden.

Beispiele 1 bis 5

| | Bsp. 1 | Bsp. 2 | Bsp. 3 | Bsp. 4 | Bsp. 5 |
|---|---|---|---|---|---|
| Sodium Carbomer | | 0,2 | | | |
| Acrylates/$C_{10}$-$C_{30}$ Alkyl Acrylate Crosspolymer | 0,3 | 0,2 | 0,6 | | |
| Hydroxypropyl Cellulose | | | | 1,0 | 1,50 |
| Xanthan Gummi | | 0,6 | 0,2 | 1,0 | 1,0 |
| Verbindung I | 3,5 | 3,5 | 1,5 | 6,0 | 5,0 |
| Dioctyl Butamidotriazon | 2,0 | 2,0 | 1,0 | | |
| Ethylhexyl Triazon | 4,0 | 4,0 | 5,0 | | |
| Aniso Triazin | 1,0 | 0,5 | | 2,0 | 2,5 |
| Bisoctyltriazol | | | | 6,0 | |
| Drometrizole Trisiloxane | | | | | |

| | Bsp. 1 | Bsp. 2 | Bsp. 3 | Bsp. 4 | Bsp. 5 |
|---|---|---|---|---|---|
| Phenylbenzmidazole Sulfonsäure | 2,0 | | | 1,0 | |
| Bisimidazylate | | | | 1,0 | |
| Terephthalylidene Dicamphor Sulfonic Acid | | | | 0,2 | |
| Ethylhexyl Methoxycinnamat | 7,5 | 10,0 | | 5,0 | |

(fortgesetzt)

| | Bsp. 1 | Bsp. 2 | Bsp. 3 | Bsp. 4 | Bsp. 5 |
|---|---|---|---|---|---|
| Octocrylen | | | | | 5,0 |
| Dimethicone-diethylbenzalmalonate | | | | 4,0 | |
| Ethylhexyl Salicylate | | | | | |
| Homosalate | | | | | |
| Butyl Methoxydibenzoylmethan | 1,0 1,0 | 1,0 1,0 | 4,0 4,0 | | |
| Titandioxid | 1,0 | 4,0 | | | |
| Zinkoxid | | | | 4,0 | |
| Caprylic/Capric Triglycerid | | | 2,0 | | |
| Hydrogenierte Coco-Glyceride | | | 3,0 | | |
| C12-15 Alkyl Benzoat | 2,0 | 2,5 | 3,0 | | |
| Dicaprylyl Ether | | 4,0 | | | |
| Butylenglycol Dicaprylat/Dicaprat | 4,0 | | 2,0 | 6,0 | |
| Dicaprylyl Carbonat | | 2,0 | | | |
| Cetyl Dimethicon | 2,0 | 0,5 | 1,0 | | |
| Shea Butter | | 2,0 | | | |
| PVP Hexadecen Copolymer | 0,5 | | 0,05 | 0,5 | |
| Glycerin | 3,0 | 7,5 | | 7,5 | 2,5 |
| Tocopherol | | 0,5 | 0,75 | | 0,2 |
| Trisodium EDTA | 1,0 | 0,5 | 0,5 | 1,0 | 1,5 |
| Natriumcitrat | | 0,2 | | | |
| Zitronensäure | | 0,1 | | 0,1 | 0,1 |
| DMDM Hydantoin | | 0,6 | | 0,2 | |
| Methylparaben | 0,5 | | 0,3 | 0,15 | |
| Phenoxyethanol | 0,5 | 0,4 | 0,4 | 1,0 | 0,60 |
| Ethanol | 3,0 | 2,0 | 3,0 | | 1,0 |
| Parfüm | 0,2 | | | 0,2 | 0,2 |
| Wasser | ad. 100 | ad. 100 | ad. 100 | ad. 100 | ad. 100 |

Beispiele 6 bis 10

| | Bsp. 6 | Bsp. 7 | Bsp. 8 | Bsp. 9 | Bsp. 10 |
|---|---|---|---|---|---|
| Sodium Carbomer | 0,5 | | | 1,5 | |
| Acrylates/$C_{10}$-$C_{30}$ Alkyl Acrylate Crosspolymer | | 0,4 | 0,1 | | 0,75 |
| Hydroxypropyl Cellulose | | | 0,5 | | 0,25 |
| Xanthan Gummi | 0,2 | 0,4 | | | |
| Verbindung I | 0,5 | 2,5 | 1,0 | 2,0 | 6,0 |

(fortgesetzt)

| | Bsp. 6 | Bsp. 7 | Bsp. 8 | Bsp. 9 | Bsp. 10 |
|---|---|---|---|---|---|
| Dioctyl Butamidotriazon | 1,0 | | 2,0 | | |
| Ethylhexyl Triazon | | 2,0 | | 2,0 | |
| Aniso Triazin | 1,0 | 0,2 | 3,0 | 1,0 | |
| Bisoctyltriazol | | | | | 8,0 |
| Drometrizole Trisiloxane | | | | | 4,0 |
| Phenylbenzmidazole Sulfonsäure | | 1,5 | | | |
| Bisimidazylate | | | 1,5 | | |
| Terephthalylidene Dicamphor Sulfonic Acid | | | | | 0,5 |
| Ethylhexyl Methoxycinnamat | | 7,5 | 5,0 | 10,0 | |
| Octocrylen | 10,0 | | 5,0 | | 5,0 |
| Dimethicone-diethylbenzalmalonate | | | | | 2,5 |
| Ethylhexyl Salicylate | | | 3,5 | 5,0 | |
| Homosalate | | | 4,0 | | |
| Butyl Methoxydibenzoylmethan | 0,5 | | | | |
| Titandioxid | 1,5 | 2,0 | 1,0 | | 2,5 |
| Zinkoxid | | | 1,0 | | 0,5 |
| Caprylic/Capric Triglycerid | | | | | |
| Hydrogenierte Coco-Glyceride | | | | | |
| C12-15 Alkyl Benzoat | | | | | 5,0 |
| Dicaprylyl Ether | | | | | 7,5 |
| Butylenglycol Dicaprylat/Dicaprat | | | | | |
| Dicaprylyl Carbonat | | 7,5 | | | |
| Cetyl Dimethicon | | | | | |
| Shea Butter | | | | | 3,0 |
| PVP Hexadecen Copolymer | 0,5 | | 0,75 | | 1,0 |
| Glycerin | 5,0 | | 10,0 | | |
| Tocopherol | 0,3 | | 1,5 | | 1,0 |
| Trisodium EDTA | 0,5 | | 0,1 | 0,5 | |
| Natriumcitrat | | | 0,3 | | |
| Zitronensäure | | | 0,15 | | |
| DMDM Hydantoin | | | | 0,3 | 0,15 |
| Methylparaben | | 0,4 | | | |
| Phenoxyethanol | | 1,0 | | | |
| Ethanol | 7,5 | | 5,0 | | 7,0 |
| Parfüm | | 0,25 | | 0,2 | |
| Wasser | ad. 100 | ad. 100 | ad. 100 | ad. 100 | ad. 100 |

Beispiele 11 bis 16

| | Bsp.11 | Bsp.12 | Bsp.13 | Bsp.14 | Bsp.15 | Bsp.16 |
|---|---|---|---|---|---|---|
| Sodium Carbomer | 0,5 | 1,5 | 1,0 | | | 0,5 |
| Acrylates/$C_{10}$-$C_{30}$ Alkyl Acrylate Crosspolymer | 1,0 | | | 0,75 | 1,0 | |
| Hydroxypropyl Cellulose | | | 0,4 | 1,0 | | 1,0 |
| Xanthan Gummi | | 0,6 | 0,2 | 1,0 | 1,0 | |
| Verbindung I | 4,0 | 0,5 | 3,0 | 2,0 | 4,0 | 1,5 |
| Dioctyl Butamidotriazon | 2,0 | 2,0 | | 2,0 | | 1,0 |
| Ethylhexyl Triazon | | 4,0 | 5,0 | 4,0 | | |
| Aniso Triazin | 1,0 | | | 1,0 | 2,5 | 1,0 |
| Bisoctyltriazol | | | 4,0 | | | |
| Drometrizole Trisiloxane | | 3,0 | | | | |
| Phenylbenzmidazole Sulfonsäure | 2,0 | | | 1,0 | | |
| Bisimidazylate | | | 1,5 | | | 3,5 |
| Terephthalylidene Dicamphor Sulfonic Acid | | | | 0,2 | | 1,0 |
| Ethylhexyl Methoxycinnamat | | 10,0 | | 5,0 | | |
| Octocrylen | 10,0 | | | | 5,0 | |
| Dimethicone-diethylbenzalmalonate | | | | 4,0 | | |
| Ethylhexyl Salicylate | | | | | | 5,0 |
| Homosalate | | | | 5,0 | | |
| Butyl Methoxy-dibenzoylmethan | 1,0 | 1,0 | 4,0 | | | 0,5 |
| Titandioxid | 1,0 | 4,0 | | | | 1,5 |
| Zinkoxid | | | | 4,0 | | |
| Caprylic/Capric Triglycerid | | | 2,0 | | | |
| Paraffinöl | | | | 1,0 | | |
| $C_{12}$-$C_{15}$ Alkyl Benzoat | 2,0 | 2,5 | 3,0 | | | |
| Dicaprylyl Ether | | 4,0 | | | | |
| Isohexadecen | 4,0 | | 2,0 | 6,0 | | |
| Dicaprylyl Carbonat | | 2,0 | | | | |
| Dibutyl Adipat | 2,0 | 0,5 | 1,0 | | | |
| Cylomethicon | | | | 3,0 | | |
| Jojobaöl | | 2,0 | | | | |
| PVP Hexadecen Copolymer | 0,5 | | 0,05 | 0,5 | | 0,5 |
| Butylen Glycol | 3,0 | 7,5 | | 7,5 | 2,5 | 5,0 |
| Ascorbyl-Palmitat | | 0,5 | 0,75 | | 0,2 | 0,3 |
| Octoxyglycerin | | 1,0 | | 0,5 | | 1,0 |
| | Bsp.11 | Bsp.12 | Bsp.13 | Bsp.14 | Bsp.15 | Bsp.16 |
| Glycin Soja | | | 2,0 | | | 1,5 |

(fortgesetzt)

| | Bsp.11 | Bsp.12 | Bsp.13 | Bsp.14 | Bsp.15 | Bsp.16 |
|---|---|---|---|---|---|---|
| Trisodium EDTA | 1,0 | 0,5 | 0,5 | | 1,5 | 0,5 |
| Natronlauge | 1,0 | 0,2 | 0,25 | | | |
| Iodopropyl Butylcarbamat | | 0,6 | | 0,2 | | |
| Phenoxyethanol | | 0,4 | | 1,0 | | |
| Ethanol | 5,0 | 2,0 | 7,0 | | | |
| Parfüm | 0,2 | | | 0,2 | 0,2 | |
| Wasser | ad. 100 | ad. 100 | ad. 100 | ad. 100 | ad. 100 | ad. 100 |

**Patentansprüche**

1. Kosmetische oder dermatologische Zubereitungen, die feindisperse Systeme vom Typ Öl-in-Wasser darstellen, enthaltend

   a) eine Ölphase,

   b) eine Wasserphase,

   c) einen oder mehrere Stabilisatoren,

   d) höchstens 2,00 Gew.-% eines oder mehrerer Emulgatoren,

   e) eine aminosubstituierte Hydroxybenzophenonverbindung der Formel I.

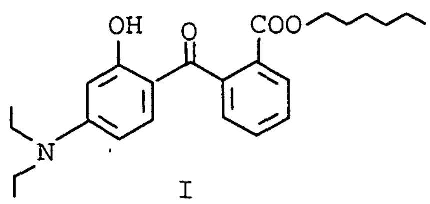

I

2. Zubereitungen nach Anspruch 1, **dadurch gekennzeichnet, dass** der Gehalt an einem oder mehreren Emulgatoren zwischen 0,5 Gew.-% und 1,5 Gew.-% ist, bezogen auf das Gesamtgewicht der Zubereitungen.

3. Zubereitungen nach einem der Ansprüche 1 oder 2, **dadurch gekennzeichnet, dass** es sich um Hydrodispersionen handelt.

4. Zubereitungen nach einem der Ansprüche 1 bis 3, **dadurch gekennzeichnet, dass** der oder die Stabilisatoren ausgewählt sind aus der Gruppe, bestehend aus Methylcellulosen, Ethylcellulosen, Xanthan, Carrageen, Copolymere aus $C_{10}$-$C_{30}$-Alkylacrylaten und einem oder mehreren Monomeren der Acrylsäure, Polyurethane und Schichtsilikate.

5. Zubereitungen nach Anspruch 4, **dadurch gekennzeichnet, dass** man als Stabilisator Ethylcellulosen verwendet.

6. Zubereitungen nach einem der Ansprüche 1 bis 5, **dadurch gekennzeichnet, dass** der Gehalt an einem oder mehreren Stabilisatoren in den Zubereitungen 0,1 bis 10 Gew.-% beträgt, bezogen auf das Gesamtgewicht der Zubereitungen.

Europäisches Patentamt

# EUROPÄISCHER RECHERCHENBERICHT

Nummer der Anmeldung
EP 02 01 9206

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| E | EP 1 240 894 A (BASF AG) 18. September 2002 (2002-09-18) * Abbildung IIA; Beispiele 6,7 * | 1-6 | A61K7/42 |
| P,A | EP 1 133 980 A (BASF AG) 19. September 2001 (2001-09-19) * Anspruch 9; Beispiele 2-13 * | 1-6 | |
| A,D | EP 1 046 391 A (BASF AG) 25. Oktober 2000 (2000-10-25) * das ganze Dokument * | 1-6 | |

RECHERCHIERTE SACHGEBIETE (Int.Cl.7)
A61K
A61Q

Der vorliegende Recherchenbericht wurde für alle Patentansprüche erstellt

| Recherchenort | Abschlußdatum der Recherche | Prüfer |
|---|---|---|
| MÜNCHEN | 11. Dezember 2002 | Vayssié, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 02 01 9206

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-12-2002

| Im Recherchenbericht angeführtes Patentdokument | | Datum der Veröffentlichung | Mitglied(er) der Patentfamilie | | | Datum der Veröffentlichung |
|---|---|---|---|---|---|---|
| EP 1240894 | A | 18-09-2002 | DE | 10113058 | A1 | 19-09-2002 |
| | | | AU | 2461302 | A | 19-09-2002 |
| | | | CN | 1382433 | A | 04-12-2002 |
| | | | EP | 1240894 | A2 | 18-09-2002 |
| | | | JP | 2002308751 | A | 23-10-2002 |
| EP 1133980 | A | 19-09-2001 | DE | 10012408 | A1 | 20-09-2001 |
| | | | AU | 2800301 | A | 20-09-2001 |
| | | | BR | 0101085 | A | 06-11-2001 |
| | | | CN | 1324610 | A | 05-12-2001 |
| | | | EP | 1133980 | A2 | 19-09-2001 |
| | | | JP | 2001261540 | A | 26-09-2001 |
| | | | US | 2002001570 | A1 | 03-01-2002 |
| EP 1046391 | A | 25-10-2000 | DE | 19917906 | A1 | 26-10-2000 |
| | | | AU | 2886900 | A | 26-10-2000 |
| | | | CN | 1273088 | A | 15-11-2000 |
| | | | EP | 1046391 | A2 | 25-10-2000 |
| | | | JP | 2000319628 | A | 21-11-2000 |
| | | | US | 6409995 | B1 | 25-06-2002 |

EPO FORM P0461

Für nähere Einzelheiten zu diesem Anhang : siehe Amtsblatt des Europäischen Patentamts, Nr.12/82